(19) Europäisches Patentamt
European Patent Office
Office européen des brevets

(11) EP 1 088 559 A2

(12) **EUROPÄISCHE PATENTANMELDUNG**

(43) Veröffentlichungstag:
**04.04.2001 Patentblatt 2001/14**

(51) Int. Cl.[7]: **A61K 49/00**, A61K 41/00

(21) Anmeldenummer: **00250324.1**

(22) Anmeldetag: **28.09.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **29.09.1999 DE 19948650**
**08.10.1999 US 158306 P**

(71) Anmelder:
**INSTITUT FÜR DIAGNOSTIKFORSCHUNG GmbH
AN DER FREIEN UNIVERSITÄT BERLIN
14050 Berlin (DE)**

(72) Erfinder:
- **Licha, Kai, Dr.
14612 Falkensee (DE)**
- **Becker, Andreas, Dr.
16761 Henningsdorf (DE)**
- **Riefke, Björn, Dr.
2808 Madrid (ES)**
- **Platzek, Johannes, Dr.
12621 Berlin (DE)**

(74) Vertreter: **Seuss, Thomas
Müllerstrasse 178
13353 Berlin (DE)**

(54) **Galenische Formulierungen**

(57) Die vorliegende Erfindung beschreibt galenische Formulierungen, welche perfluoralkylhaltige Farbstoffmoleküle und andere perfluoralkylhaltige Substanzen enthalten. Die neuen Formulierungen sind unter anderem als Kontrastmittel für die Nahinfrarotdiagnostik geeignet.



Printed by Xerox (UK) Business Services
2.16.7 (HRS)/3.6

**Beschreibung**

Die Erfindung liegt auf dem Gebiet der galenischen Formulierungen, welche insbesondere als Kontrastmittel zur Darstellung der Lymphknoten eingesetzt werden. Die Erfindung betrifft den in den Patentansprüchen gekennzeichneten Gegenstand, nämlich neue galenische Formulierungen, welche perfluoralkylhaltige Farbstoffmoleküle und andere perfluoralkylhaltige Substanzen enthalten.

Maligne Tumoren metastasieren gehäuft in regionale Lymphknoten, wobei auch mehrere Lymphknotenstationen beteiligt sein können. So werden Lymphknotenmetastasen in etwa 50 - 69% aller Patienten mit malignen Tumoren gefunden (Elke, Lymphographie, in: Frommhold, Stender, Thurn (eds.), Radiologische Diagnostik in Klinik und Praxis, Band IV, Thieme Verlag Stuttgart, 7th ed., 434-496, 1984). Die Diagnose eines metastatischen Befalls von Lymphknoten ist im Hinblick auf die Therapie und Prognose maligner Erkrankungen von großer Bedeutung. Mit den modernen bildgebenden Methoden (CT, US und MRT) werden lymphogene Absiedlungen von malignen Tumoren nur unzureichend erkannt, da zumeist nur die Größe sowie die Form des Lymphknotens als Diagnosekriterium herangezogen werden kann. Damit sind kleine Metastasen in nicht-vergrößerten Lymphknoten (< 2 cm) nicht von Lymphknotenhyperplasien ohne malignen Befall zu unterscheiden (Steinkamp et al., Sonographie und Kernspintomographie: Differentialdiagnostik von reaktiver Lymphknotenvergrößerung und Lymphknotenmetastasen am Hals, Radiol.diagn. 33:158, 1992).

Es wäre wünschenswert, Lymphknoten mit metastatischem Befall und hyperplastische Lymphknoten mit Hilfe spezifischer Kontrastmittel zu unterscheiden. Dabei könnte das Kontrastmittel intravasal oder interstitiell/intrakutan appliziert werden (s.a. Siefert, H.M. et al., Lymphology 13, 150 — 157, 1980). Die Applikation interstitiell/intrakutan hat den Vorteil, daß die Substanz direkt vom streuenden Herd (z.B. Primärtumor) durch die entsprechenden Lymphwege in die potentiell betroffenen, regionalen Lymphknotenstationen transportiert wird. Gleichfalls kann mit einer geringen Dosis eine hohe Konzentration des Kontrastmittels in den Lymphknoten erreicht werden. Solche interstitiell zu verabreichenden Marker werden vor allem in der nuklearmedizinischen Beurteilung verwendet (mittels radioaktiver Partikel wie z.B. $^{198}$Au-Kolloid). Nuklearmedizinische Methoden haben aber nur eine sehr ungenügende räumliche Auflösung, im Unterschied zur Kernspintomographie mit ihrer hohen räumlichen Auflösung im Bereich von Bruchteilen von Millimetern. Die direkte Röntgen-Lymphographie (Injektion einer öligen Kontrastmittelsuspension in ein präpariertes Lymphgefäß) ist eine nur noch selten benutzte, invasive Methode, die nur wenige Lymphabflußstationen darstellen kann. Experimentell werden in Tierexperimenten auch fluoreszenzmarkierte Dextrane verwendet, um nach deren interstitiellen Applikation den Lymphabfluß beobachten zu können. Allen gebräuchlichen Markern für die Darstellung von Lymphwegen und Lymphknoten nach interstitieller/intrakutaner Applikation ist also gemein, daß es sich um Substanzen mit partikulärem Charakter ("particulates", z.B. Emulsionen und Nanokristallsuspensionen) oder große Polymere handelt (s.a. WO 90/14846). Die bisher beschriebenen Zubereitungen erwiesen sich jedoch aufgrund ihrer mangelnden lokalen und systemischen Verträglichkeit sowie ihrer geringen Lymphgängigkeit, die eine unzureichende diagnostischen Effizienz bedingt, zumeist für die indirekte Lymphographie als ungeeignet.

Insgesamt besteht daher ein großer Bedarf an lymphspezifischen Kontrastmitteln mit geeigneten pharmazeutischen sowie pharmakologischen Eigenschaften. Bei den pharmazeutischen Eigenschaften stehen zunächst die möglichst hohe Kontrastmittelbeladung sowie eine ausreichende Stabilität im Vordergrund. Bei den pharmakologischen Eigenschaften steht neben einer diagnostisch relevanten und möglichst gleichmäßigen Lymphanreicherung über mehrere (bzw. bei intravenöser Applikation über alle) Lymphstationen hinweg vor allem eine rasche und vollständige Ausscheidung des Kontrastmittels, zur Vermeidung einer unnötigen Belastung des Gesamtorganismus, im Vordergrund. Darüber hinaus müssen entsprechende Zubereitungen über eine ausreichende lokale und akute Verträglichkeit verfügen.

Hinsichtlich der Anwendung in der radiologischen Praxis ist neben einer möglichst einfachen Anwendbarkeit entsprechender Zubereitungen deren rascher "Wirkungseintritt" von zentraler Bedeutung. So sollte möglichst bereits innerhalb weniger Stunden nach der Kontrastmittelapplikation eine Bildgebung möglich sein.

Kontrastmittel, die zur Darstellung der Lymphknoten in der Kernspintomographie geeignet sind, werden in der deutschen Offenlegungsschrift DE 196 03 033 beschrieben. Dort sind perfluoralkylhaltige Metallkomplexe offenbart, die bevorzugt als Lymphographika eingesetzt werden (siehe Abbildung 1 der DE 196 03 033). In der deutschen Offenlegungsschrift DE 197 29 013 werden ähnliche Metallkomplexe beschrieben, die insbesondere als blood-pool-agents geeignet sind.

In der internationalen Anmeldung WO 96/17628 ist ein Verfahren zur In-vivo-Diagnostik mittels NIR-Strahlung beschrieben. Derartige diagnostische Verfahren befinden sich zur Zeit in der Entwicklung. Die in diesem Dokument beschriebenen Kontrastmittel sind nicht zur Darstellung der Lymphknoten geeignet. Es besteht daher ein Bedarf an geeigneten lymphspezifischen Kontrastmitteln für diese neuen Diagnoseverfahren.

Aufgabe der Erfindung ist es daher, neue galenische Formulierungen zur Verfügung zu stellen, die als Kontrastmittel insbesondere für die Darstellung der Lymphknoten in den obengenannten neuen Diagnostizierverfahren geeignet sind und die die genannten pharmazeutischen und pharmakologischen Anforderungen erfüllen.

Diese Aufgabe wird durch die galenischen Formulierungen der vorliegenden Erfindung gelöst.

Die neuen galenischen Formulierungen enthalten perfluoralkylhaltige Farbstoffmoleküle, die als Kontrastmittel in der Nahinfrarotdiagnostik eingesetzt werden können. Die Farbstoffe erfüllen bestimmte photophysikalische und chemische Anforderungen. Sie besitzen hohe Absorptionskoeffizienten und hohe Fluoreszenzquantenausbeuten, um ein effektives Signal auch bei geringsten Gewebekonzentrationen zu erzeugen. Die Absorptionsmaxima überdekken frei wählbar einen weiten spektralen Bereich. Für die Detektion in tieferen Gewebeschichten (mehrere Zentimeter unter der Oberfläche) ist der Spektralbereich zwischen 600 und 900 nm essentiell, während für oberflächliche Detektion Absorptionswellenlängen von 400 bis 600 nm ausreichen. Weiter besitzen die Farbstoffe eine hohe chemische Stabilität und Photostabilität.

Das wesentliche Problem bei Nutzung von Licht zur Fluoreszenzanregung ist die begrenzte Eindringtiefe des Lichtes, die im VIS im Submillimeterbereich liegt, im NIR jedoch Zentimeter betragen kann. Hinsichtlich der Eindringtiefe unproblematisch sind Detektionsverfahren in oberflächlichen Gewebeerkrankungen, sowie weichen Geweben. Da eine Vielzahl von Gewebeveränderungen (z. B. Brusttumoren, Hauttumoren, Lymphknotenveränderungen) oberflächlich lokalisiert sind, bieten sich optische Diagnoseverfahren zusätzlich zu den konventionellen Methoden an, um eine Gewebedifferenzierung anhand unterschiedlicher Absorptions- und Fluoreszenzmuster durchzuführen. Die ausgeprägte Lichtstreuung, die mit wachsender Gewebedicke zunehmend an Einfluß gewinnt, verringert dabei sowohl die Auflösung als auch den Kontrast eines optischen Bildes. Farbstoffe, die als sog. optische Kontrastmittel eingesetzt werden und sich in den zu detektierenden Geweben anreichern, können prinzipiell zu einer Erhöhung des diagnostisches Wertes optischer Detektionsverfahren führen, in dem sie die Absorption des Gewebes erhöhen und mit der farbstoffspezifischen Fluoreszenz ein zusätzliches Meßsignal liefern, das über einem geringen Gewebehintergrund mit hoher Sensitivität beliebig oft detektiert werden kann.

Bevorzugt sind Farbstoffe aus den folgenden Klassen:

- Polymethinfarbstoffe, wie z. B. Cyaninfarbstoffe, Squariliumfarbstoffe, Croconiumfarbstoffe, Oxonolfarbstoffe, Merocyaninfarbstoffe, Kryptocyaninfarbstoffe;
- Xanthinfarbstoffe, wie z. B. Fluorescein und Rhodamin und dessen Derivate
- Heteroaromatische, kationische Farbstoffe, wie z.B. Oxazine, Phenoxazine, Thiazine, Phenothiazine

Die neuen perfluoralkylhaltigen Farbstoffmoleküle sind Verbindungen der allgemeinen Formel I

$$R_f — L — A \qquad (I)$$

worin $R_f$ einen geradkettigen oder verzweigten Perfluoralkylrest mit 4 bis 30 Kohlenstoffatomen darstellt, L für einen Linker und A für ein Farbstoffmolekül steht.

Der Linker L ist eine direkte Bindung oder eine geradkettige oder verzweigte Kohlenstoffkette mit bis zu 20 Kohlenstoffatomen, welche mit ein oder mehreren —OH, $-COO^-$, $-SO_3$-Gruppen substituiert sein kann und/oder gegebenenfalls durch eine oder mehrere —O-, -S-, -CO-, -CONH-, -NHCO-, -CONR-, -NRCO-, $-SO_2$-, -NH-, -NR-Gruppen oder ein Piperazin unterbrochen ist, wobei R für einen $C_1$- bis $C_{10}$-Alkylrest steht, welcher gegebenenfalls mit einer oder mehreren OH-Gruppen substituiert und/oder durch ein oder mehrere Sauerstoffatome unterbrochen ist.
Das Farbstoffmolekül A ist ein Farbstoff aus der Klasse der Polymethinfarbstoffe, Xanthinfarbstoffe oder der heteroaromatischen, kationischen Farbstoffe. Bevorzugt ist das Farbstoffmolekül A ein Cyaninfarbstoff, Sqariliumfarbstoff, Croconiumfarbstoff, Oxonolfarbstoff, Merocyaninfarbstoff, Kryptocyaninfarbstoff, Fluoresceinfarbstoff, Rhodaminfarbstoff, Oxazinfarbstoff, Phenoxazinfarbstoff, Thiazinfarbstoff oder Phenothiazinfarbstoff. Besonders bevorzugt ist das Farbstoffmolekül A ein Molekül gemäß der allgemeinen Formel II :

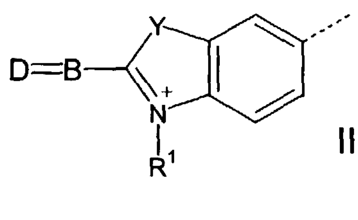

II

worin

D für ein Fragment entsprechend den allgemeinen Formeln III bis VI steht, wobei die mit einem Stern gekennzeichnete Position die Verknüpfung mit B bedeutet:

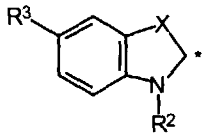 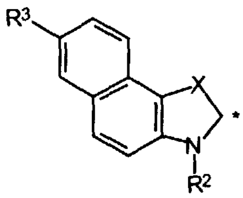 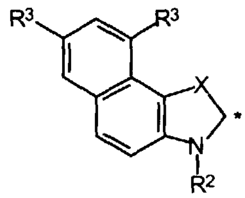 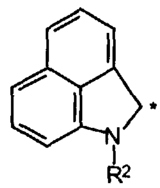

III IV V VI

und worin B für ein Fragment entsprechend den allgemeinen Formeln VII bis XII steht:

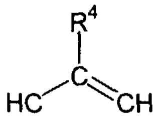 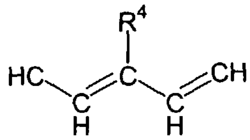 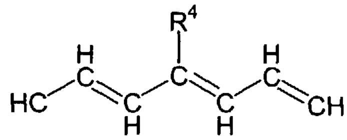

VII VIII IX

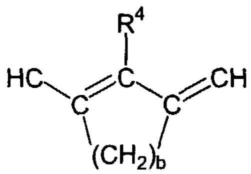 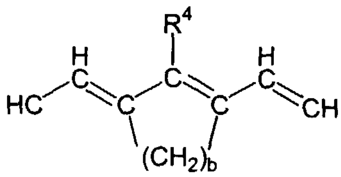 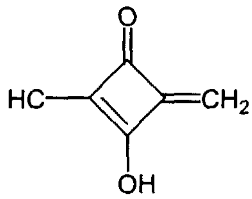

X XI XII

wobei $R^1$ und $R^2$ eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$- $C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

$R^3$ für einen Rest $-COOE^1$, $-CONE^1E^2$, $-NHCOE^1$, $-NHCONHE^1$, $-NE^1E^2$, $- OE^1$, $-OSO_3E^1$, $-SO_3E^1$, $-SO_2NHE^1$, $-E^1$ steht,

wobei $E^1$ und $E^2$ unabhängig voneinander ein Wasserstoffatom, eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$- $C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

und wobei $R^4$ für ein Wasserstoffatom, ein Fluor-, Chlor-, Brom-, Iodatom oder eine verzweigte oder geradkettige $C_1$-$C_{10}$-Alkylkette steht,

b eine Zahl 2 oder 3 bedeutet,

sowie X und Y unabhängig voneinander O, S, -CH=CH- oder $C(CH_3)_2$ bedeuten.

Besonders bevorzugte perfluoralkylhaltige Farbstoffmoleküle enthalten einen Perfluoralkylrest $R_f$ mit 6 bis 12 Kohlenstoffatomen, einen Linker L, welcher aus einer $C_1$-$C_{10}$-Alkylgruppe besteht, die ein oder mehrere Sauerstoffatome und/oder eine oder mehrere —CONH-, -NHCO-, -CONR-, -NRCO-Gruppen enthält, worin R für einen $C_1$-$C_5$-Alkylrest steht, welcher mit ein oder mehreren OH-Gruppen substituiert sein kann, und als Farbstoffmolekül einen Cyaninfarbstoff. Unter den Cyaninfarbstoffen sind Indocarbocyaninfarbstoffe, Indodicarbocyaninfarbstoffe und Indotricarbocyaninfarbstoffe bevorzugt. Besonders bevorzugte Farbstoffmoleküle sind die folgenden Verbindungen:

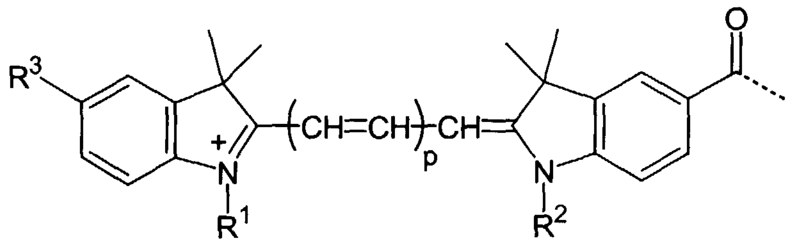

XIII

worin

p für 1, 2 oder 3,
$R^1$ und $R^2$ unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
$R^3$ für Wasserstoff, für einen Rest $-COOE^1$, $-CONE^1E^2$, $-NHCOE^1$, $-NHCONHE^1$, $-NE^1E^2$, $-OE^1$, $-OSO_3E^1$, $-SO_3E^1$, $-SO_2NHE^1$ steht,
wobei $E^1$ und $E^2$ unabhängig voneinander für ein Wasserstoffatom oder für einen Methyl-, Ethyl- oder einen $C_3$-$C_6$-Alkylrest, der von 0 bis 2 Sauerstoffatomen und/oder von 0 bis 1 Carbonylgruppen unterbrochen ist und/oder mit 0 bis 5 Hydroxygruppen substituiert ist, oder für einen Poly(oxyethylen)glykolrest mit 2 bis 30 $-CH_2CH_2O$-Einheiten stehen.

Weiter enthalten die erfindungsgemäßen galenischen Formulierungen andere perfluoralkylhaltige Verbindungen, z.B. perfluoralkylhaltige Metallkomplexe. Perfluoralkylhaltige Metallkomplexe und ihre Herstellung wurden bereits in den deutschen Offenlegungsschriften DE 196 03 033, DE 197 29 013 und WO 97/26017 beschrieben. Diese perfluoralkylhaltigen Metallkomplexe sind Verbindungen der allgemeinen Formel XIV

$$R_f — M \qquad (XIV)$$

worin $R_f$ einen geradkettigen oder verzweigten Perfluoralkylrest mit 4 bis 30 Kohlenstoffatomen darstellt und M ein Molekülteil ist, das 1 - 6 Metallkomplexe enthält.

Das Molekülteil M steht beispielsweise für eine Gruppe L — $M^1$, wobei L für einen Linker und $M^1$ für einen Metallkomplex mit einem offenkettigen oder cyclischen Chelatoren steht, welcher als Zentralatom ein Atom der Ordnungszahlen 21 — 29, 39, 42, 44 oder 57 — 83 enthält. Der Linker L ist dabei eine direkte Bindung, eine Methylengruppe, eine - NHCO-Gruppe, eine Gruppe

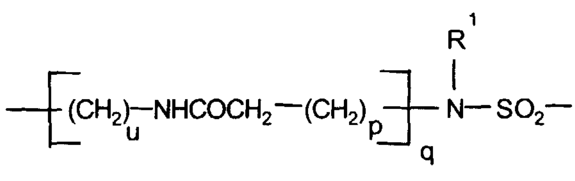

,

wobei $p^1$ die Zahlen 0 bis 10, q und u unabhängig voneinander die Zahlen 0 oder 1 und

$R^1$ ein Wasserstoffatom, eine Methylgruppe, eine $-CH_2-OH$-Gruppe, eine $-CH_2-CO_2H$-Gruppe oder eine $C_2$-$C_{15}$-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 $C_1$-$C_4$-Alkoxygruppen, 1 bis 2 Carboxygruppen,

oder eine geradkettige, verzweigte, gesättigte oder ungesättigte $C_2$-$C_{30}$-Kohlenstoffkette ist, die gegebenenfalls 1 bis 10 Sauerstoffatome, 1 bis 3 - $NR^1$-Gruppen, 1 bis 2 Schwefelatome, ein Piperazin, eine $-CONR^1$-Gruppe, eine $-NR^1CO$-Gruppe, eine $-SO_2$-Gruppe, eine $-NR^1-CO_2$-Gruppe, 1 bis 2 -CO-Gruppen, eine Gruppe

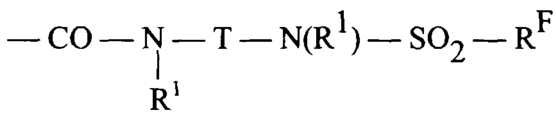

oder 1 bis 2 gegebenenfalls substituierte Aryle enthält und/oder durch diese Gruppen unterbrochen ist, und/oder gegebenenfalls substituiert ist mit 1 bis 3 $-OR^1$-Gruppen, 1 bis 2 Oxogruppen, 1 bis 2 $-NH-COR^1$-Gruppen, 1 bis 2 $-CONHR^1$-Gruppen, 1 bis 2 $-(CH_2)_p-CO_2H$-Gruppen, 1 bis 2 Gruppen $-(CH_2)_p-(O)_q-CH_2CH_2-R^F$, wobei

$R^1$, $R^F$ und p und q die oben angegebenen Bedeutungen haben und

T eine $C_2$-$C_{10}$-Kette bedeutet, die gegebenenfalls durch 1 bis 2 Sauerstoffatome oder 1 bis 2 -NHCO-Gruppen unterbrochen ist.

Der Metallkomplex $M^1$ steht dabei für die folgenden Metallkomplexe:

- einen Komplex der allgemeinen Formel XV

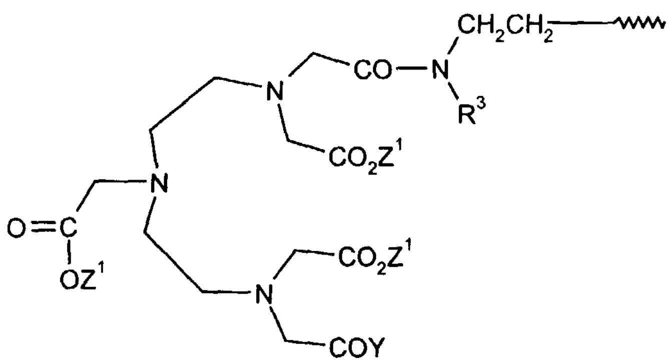

(XV)

in der $R^3$, $Z^1$ und Y unabhängig voneinander sind und

$R^3$ die Bedeutung von $R^1$ hat oder $-(CH_2)_m-L-R^F$ bedeutet, wobei m 0, 1 oder 2 ist und L und $R^F$ die o.g. Bedeutung haben,

$Z^1$ unabhängig voneinander ein Wasserstoffatom oder ein Metallionenäquivalent der Ordnungszahlen 21 - 29, 39, 42, 44 oder 57 - 83 bedeutet,

Y $-OZ^1$ oder

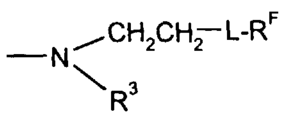

oder

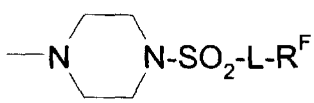

bedeutet, wobei $Z^1$, L, $R^F$ und $R^3$ die o. g. Bedeutungen haben.

- einen Komplex der allgemeinen Formel XVI

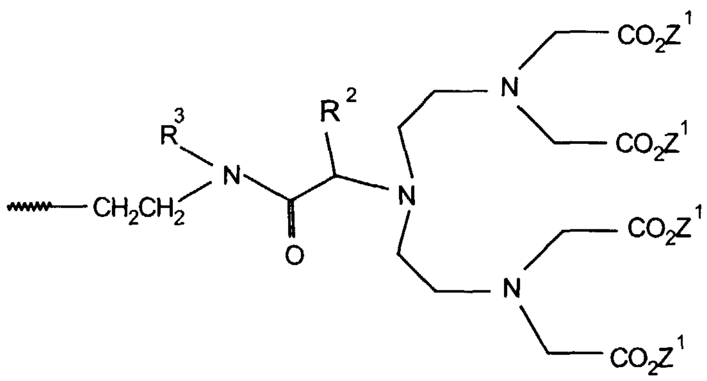

(XVI)

in der $R^3$ und $Z^1$ die oben genannten Bedeutungen aufweisen und $R^2$ die Bedeutung von $R^1$ hat.

- einen Komplex der allgemeinen Formel XVII

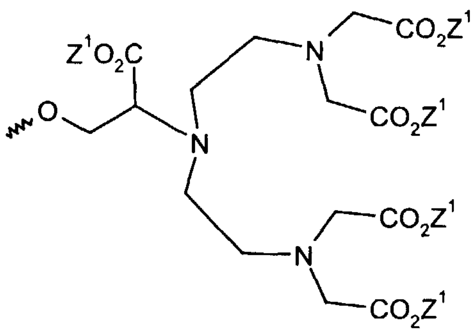

(XVII)

in der $Z^1$ die oben genannte Bedeutung hat.

- einen Komplex der allgemeinen Formel XVIII

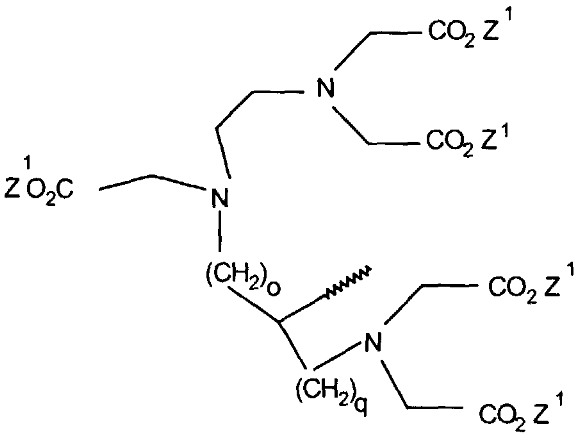

(XVIII)

in der $Z^1$ die oben genannte Bedeutung hat und o und q für die Ziffern 0 oder 1 stehen und die Summe $o + q = 1$ ergibt.

- einen Komplex der allgemeinen Formel XIX

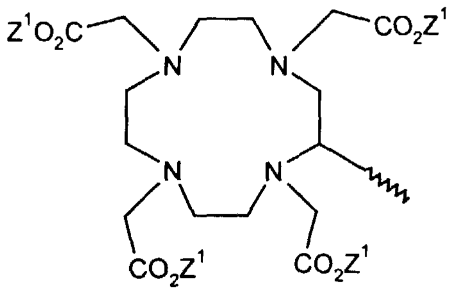

(XIX)

in der $Z^1$ die oben genannte Bedeutung hat.

- einen Komplex der allgemeinen Formel XX

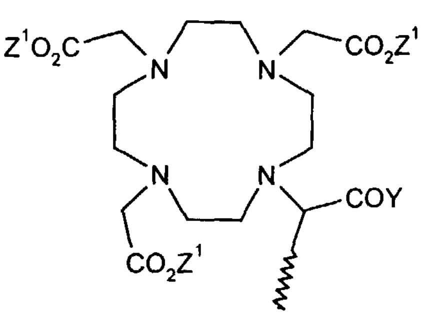

(XX)

in der $Z^1$ und Y die oben genannten Bedeutungen haben.

- einen Komplex der allgemeinen Formel XXI

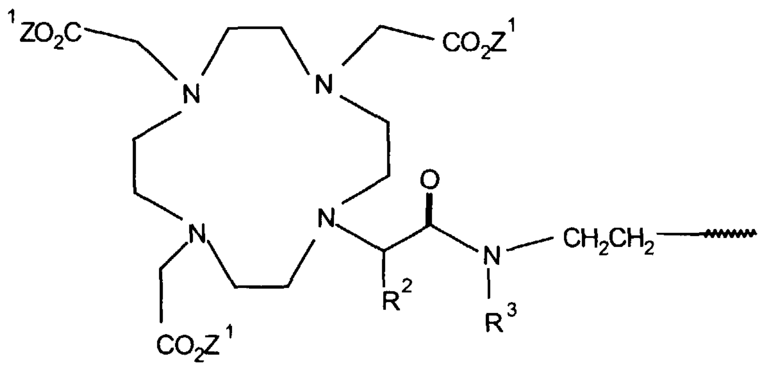

(XXI)

in der $R^3$ und $Z^1$ die oben genannten Bedeutungen haben und $R^2$ die o.g. Bedeutung von $R^1$ hat.

- einen Komplex der allgemeinen Formel XXII

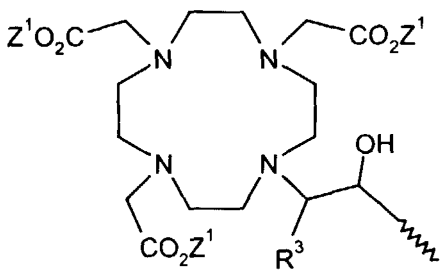

(XXII)

in der $R^3$ und $Z^1$ die oben genannten Bedeutungen haben.

- einen Komplex der allgemeinen Formel XXIII

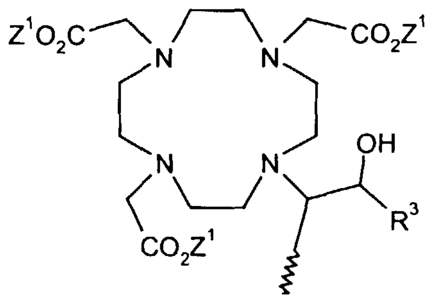

(XXIII)

in der $R^3$ und $Z^1$ die oben genannten Bedeutungen haben.

- einen Komplex der allgemeinen Formel XXIV

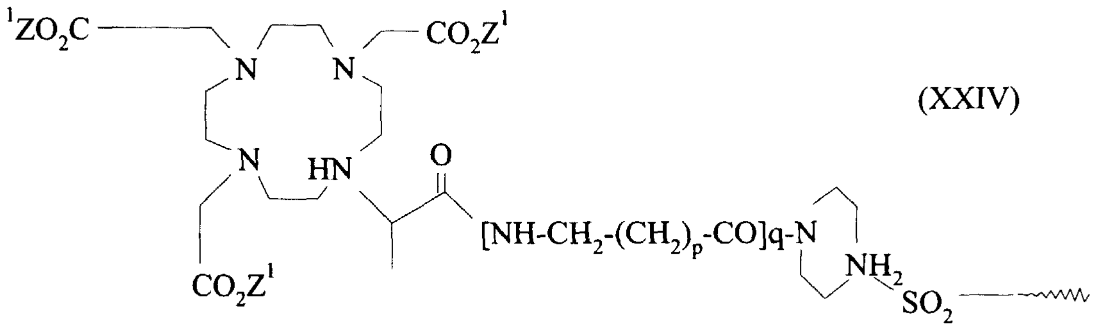

(XXIV)

in der $Z^1$ p und q die oben genannte Bedeutung haben und $R^2$ die Bedeutung von $R^1$ hat.

- einen Komplex der allgemeinen Formel XXV

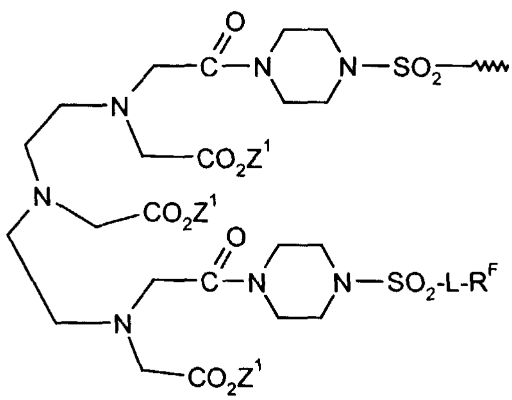

(XXV)

in der L· $R^F$ und $Z^1$ die oben genannten Bedeutungen haben.

- einen Komplex der allgemeinen Formel XXVI

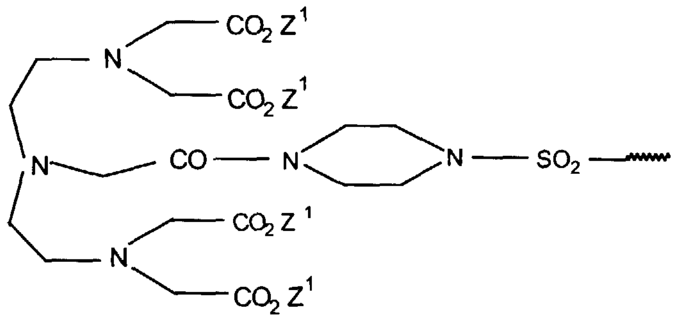

(XXVI)

in der $Z^1$ die oben genannte Bedeutung hat.

Derartige Verbindungen und deren Herstellung sind in der deutschen Offenlegungsschrift DE 196 03 033 A1 und in der Internationalen Patentanmeldung WO 97/26017 beschrieben.

Weiter kann das Molekülteil M gemäß Formel XIV die folgende Struktur aufweisen:

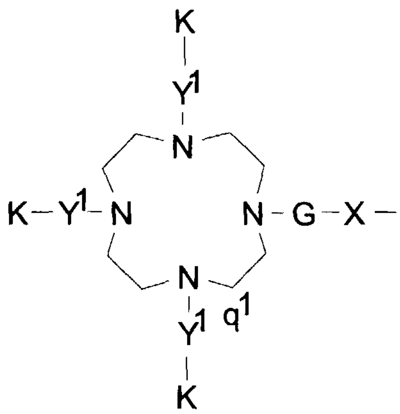

wobei

- $q^1$ eine Zahl 0,1,2 oder 3 ist,
- K für einen Komplexbildner oder Metallkomplex oder deren Salze organischer und/oder anorganischer Basen oder Aminosäuren oder Aminosäureamide steht,
- X eine direkte Bindung zur Perfluoralkylgruppe, eine Phenylengruppe oder eine $C_1$-$C_{10}$-Alkylenkette ist, die gegebenenfalls 1 - 15 Sauerstoff-, 1 - 5 Schwefelatome, 1 - 10 Carbonyl-, 1 - 10 (NR)-, 1 - 2 $NRSO_2$-, 1 - 10 CONR-, 1 Piperidin-, 1 - 3 $SO_2$-, 1 - 2 Phenylengruppen enthält oder gegebenenfalls durch 1 - 3 Reste $R^F$ substituiert ist, worin R für ein Wasserstoffatom, eine Phenyl-, Benzyl- oder eine $C_1$-$C_{15}$-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1 - 5 Methoxy-, 1 - 3 Carboxy-, 1 - 3 $R^F$-Reste substituiert ist
- $Y^1$ eine direkte Bindung oder eine Kette der allgemeinen Formel XXVII oder XXVIII ist:

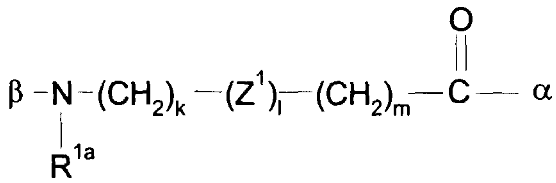

(XXVII)

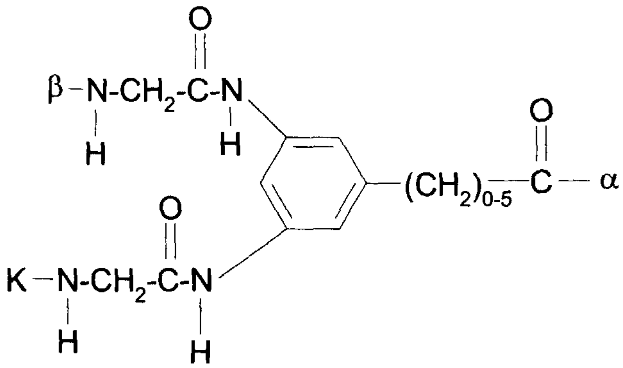

(XXVIII)

worin

- $R^{1a}$ ein Wasserstoffatom, eine Phenylgruppe, eine Benzylgruppe oder eine $C_1$-$C_7$ Alkylgruppe ist, die gegebenenfalls substituiert ist mit einer Carboxy-, einer Methoxy- oder einer Hydroxygruppe,
- $Z^1$ eine direkte Bindung, eine Polyglycolethergruppe mit bis zu 5 Glycoleinheiten oder ein Molekülteil der allgemeinen Formel XXIX ist

$$—CH(R^{2a})- \qquad (XXIX)$$

worin $R^{2a}$ eine $C_1$-$C_7$-Carbonsäure, eine Phenylgruppe, eine Benzylgruppe oder eine $-(CH_2)_{1-5}$-NH-K-Gruppe ist,
- $\alpha$ die Bindung an das Stickstoffatom der Gerüstkette, $\beta$ die Bindung zum Komplexbildner oder Metallkomplex K darstellt,
- und in der die Variablen k und m für natürliche Zahlen zwischen 0 und 10 und 1 für 0 oder 1 stehen,

und wobei

- G eine CO- oder $SO_2$-Gruppe ist.

Derartige Verbindungen und deren Herstellung sind in der deutschen Offenlegungsschrift DE 197 29 013 A1 beschrieben.

Weiter kann das Molekülteil A gemäß allgemeiner Formel I für eine Gruppe $L^1$-$M^2$ stehen, worin $L^1$ für einen Linker und $M^2$ für einen Metallkomplex steht. Der Linker $L^1$ ist dabei ein Molekülteil gemäß allgemeiner Formel XXX

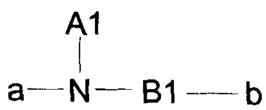

(XXX),

worin

N ein Stickstoffatom darstellt,

A1 ein Wasserstoffatom, eine geradkettige oder verzweigte $C_1$-$C_{30}$-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 -$OR^4$-Gruppen, mit $R^4$ in der Bedeutung eines Wasserstoffatoms oder eines $C_1$-$C_7$-Alkylrestes, oder -B1-$R^F$ bedeutet,

B1 eine geradkettige oder verzweigte $C_1$-$C_{30}$-Alkylengruppe, die gegebenenfalls unterbrochen ist durch 1-10 Sauerstoffatome, 1-5 -NH-CO- Gruppen, 1-5 -CO-NH- Gruppen, durch eine (gegebenenfalls durch eine COOH-Gruppe substituierte) Phenylengruppe, 1-3 Schwefelatome, 1-2 -N(B2)-$SO_2$- Gruppen, und/oder 1-2 -$SO_2$-N(B2)-Gruppen mit B2 in der Bedeutung von A1, eine NHCO-Gruppe, eine CONH-Gruppe, eine N(B2)-$SO_2$-Gruppe, oder eine -$SO_2$-N(B2)- Gruppe und/oder gegebenenfalls substituiert ist mit dem Rest $R^F$,
bedeutet,

und worin a die Bindung zum Metallkomplex $M^2$ und b die Bindung zur Perfluoralkylgruppe $R^F$ darstellt.

Der Metallkomplex $M^2$ steht dabei für einen Metallkomplex der allgemeinen Formel XXXI

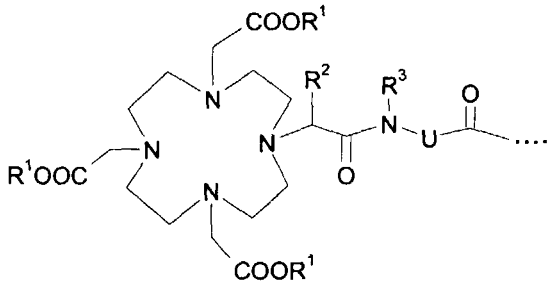

(XXXI)

wobei $R^1$ für ein Wasserstoffatom oder ein Metallionenäquivalent der Ordnungszahlen 21-29, 31, 32, 37-39, 42-44, 49 oder 57-83, $R^2$ und $R^3$ für ein Wasserstoffatom, eine $C_1$-$C_7$-Alkylgruppe, eine Benzylgruppe, eine Phenylgruppe, -$CH_2OH$ oder -$CH_2$-$OCH_3$, U für den Rest L, wobei aber L und U unabhängig voneinander gleich oder verschieden sein können, stehen.

Derartige Verbindungen und deren Herstellung werden in der deutschen Patentanmeldung mit dem Aktenzeichen 199 14 101.0 sowie in den nachfolgenden Beispielen beschrieben.

Besonders bevorzugt sind Metallkomplexe, bei denen das Zentralatom ein Gadoliniumatom (Ordnungszahl 64) ist. Metallkomplexe mit cyclischen Chelatoren sind gegenüber denen mit offenkettigen Chelatoren bevorzugt.

Besonders bevorzugte Gadoliniumkomplexe sind der Gadoliniumkomplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan (Herstellung siehe WO 97/26017, Beispiel 33),

der Gadoliniumkomplex von 10-[2-Hydroxy-4-aza-5-oxo-7-oxa-10,10,11,11,12,12,13,13,14,14,15,15,16,16,17,17,17-heptadecafluorheptadecyl]-1,4,7-tris(carboxymethyl)-1,4,7,10-tetraazacyclododecan (Herstellung siehe DE 196 03 033, Beispiel 2),

1,4,7-Tris{1,4,7-tris(N-carboxylatomethyl)-10-(N-1-methyl-3,6-diaza-2,5,8-trioxooctan-1,8-diyl)-1,4,7,10-tetraazacyclododecan, Gd-Komplex}-10-(N-2H,2H,4H,4H,5H,5H-3-oxa-perfluor-tridecanoyl)-1,4,7,10-tetraazacyclododecan (Herstellung siehe DE 197 29 013, Beispiel 1),

1,4,7-Tris{1,4,7-tris[(N-carboxylatomethyl)]-10-[N-1-methyl-3-aza-2,5-dioxopentam-1,5-diyl]-1,4,7,10-tetraazacyclododecan, Gd-Komplex}-10-[2-(N-ethyl-N-perfluoroctylsulfonyl)-amino]-acetyl-1,4,7,10-tetraazacyclododecan (Herstellung siehe DE 197 29 013, Beispiel 12),

der Gadolinium-Komplex von 10-[2-Hydroxy-4-aza-5-oxo-7-aza-7(perfluoroctylsulfonyl)-nonyl]-1,4,7-tris(carboxymethyl)-1,4,7,10-tetraazacyclododecan (Herstellung siehe DE 196 03 033, Beispiel 1),

1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex (Herstellung siehe Beispiele),

1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex (Herstellung siehe Beispiele),

1,4,7-Tris(carboxylatomethyl)-10-{(3-aza-4-oxo-hexan-5-yl)-säure-[N-3,6,9,12,15-pentaoxa)-hexadexyl)-N-(1H, 1H,

2H, 2H, 4H, 4H, 5H, 5H-3-oxa)-perfluortridecyl]-amid}-1,4,7,10-tetraazacyclododecan, Gadoliniumkomplex (Herstellung siehe Beispiele), sowie 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex (Herstellung siehe Beispiele).

Statt der perfluoralkylhaltigen Metallkomplexe können auch andere perfluoralkylhaltige Verbindungen in den erfindungsgemäßen galenischen Formulierungen enthalten sein. Derartige Verbindungen sind Verbindungen der allgemeinen Formel XXXII

$$R_f — L^2 — G^1 \qquad (XXXII)$$

worin $R_f$ einen geradkettigen oder verzweigten Perfluoralkylrest mit 4 bis 30 Kohlenstoffatomen darstellt, $L^2$ für einen Linker und $G^1$ für eine hydrophile Gruppe steht.
Der Linker $L^2$ ist eine direkte Bindung, eine —$SO_2$-Gruppe oder eine geradkettige oder verzweigte Kohlenstoffkette mit bis zu 20 Kohlenstoffatomen, welche mit ein oder mehreren —OH, -$COO^-$, -$SO_3$-Gruppen substituiert sein kann und/oder gegebenenfalls ein oder mehrere —O-, -S-, -CO-, -CONH-, -NHCO-, -CONR"-, -NR"CO-, -$SO_2$-, -$PO_4^-$-, - NH-, -NR"-Gruppen, einen Arylring oder ein Piperazin enthält, wobei R" für einen $C_1$-bis $C_{20}$-Alkylrest steht, welcher wiederum ein oder mehrere O-Atome enthalten kann und/oder mit —$COO^-$ oder $SO_3$-Gruppen substituiert sein kann. Die hydrophile Gruppe $G^1$ steht für ein Mono- oder Disaccharid, eine oder mehrere benachbarte —$COO^-$ oder —$SO_3$-Gruppen, eine Dicarbonsäure, eine Isophthalsäure, eine Picolinsäure, eine Benzolsulfonsäure, eine Tetrahydropyrandicarbonsäure, eine 2,6-Pyridindicarbonsäure, ein quartäres Ammoniumion, eine Aminopolycarbonsäure, eine Aminodipolyethylenglycolsulfonsäure, eine Aminopolyethylenglycolgruppe, eine $SO_2$-$(CH_2)_2$-OH-Gruppe, eine Polyhydroxyalkylkette mit mindestens zwei Hydroxylgruppen der eine oder mehrere Polyethylenglycolketten mit mindestens zwei Glycoleinheiten, wobei die Polyethylenglycolketten durch eine —OH oder —$OCH_3$-Gruppe terminiert sind.
Derartige Substanzen sind bereits bekannt (siehe z.B. Tetrahedron Letters, Vol. 36, No. 4, pp. 539 — 542, 1995). Die Synthese einiger dieser Verbindungen wird detailliert in den nachfolgenden Beispielen beschrieben. Bevorzugt werden solche Verbindungen verwendet, die als hydrophile Gruppe $G^1$ ein Monosaccharid enthalten.

Besonders bevorzugte perfluoralkylhaltige Verbindungen enthalten einen Perfluoralkylrest $R_f$mit 6 bis 12 Kohlenstoffatomen, einen Linker $L^2$, welcher eine —$SO_2$-Gruppe oder eine geradkettige oder verzweigte Kohlenstoffkette mit bis zu 20 Kohlenstoffatomen darstellt, die wiederum ein oder mehrere —O-, -CO-, -CONH-, - NHCO-, -CONR"-, -NR"CO-, -$SO_2$-Gruppen oder ein Piperazin enthält, worin R" die oben angegebene Bedeutung hat, und ein Monosaccharid als hydrophile Gruppe $G^1$.

Es ist auch möglich, galenische Formulierungen mit drei Komponenten herzustellen und diese als Kontrastmittel zur Darstellung der Lymphknoten zu benutzen. Derartige Formulierungen sind detailliert in den nachfolgenden Beispielen beschrieben.

Die erfindungsgemäßen Substanzmischungen können in einem Lösungsmittel gelöst vorliegen. Das Lösungsmittel ist bevorzugt Wasser. Der Anteil der perfluoralkylhaltigen Farbstoffmoleküle beträgt zwischen 0.1 und 10 mol% bezogen auf die Gesamtmenge an perfluoralkylhaltigen Substanzen, bevorzugt zwischen 1 und 10 mol%. Bevorzugt sind Mischungen aus perfluoralkylhaltigen Farbstoffmolekülen und anderen perfluoralkylhaltigen Verbindungen, bei denen die Perfluoralkylketten eine Länge von 6 bis 12 Kohlenstoffatomen haben. Besonders bevorzugt sind Mischungen, bei denen sowohl die perfluoralkylhaltigen Farbstoffmoleküle als auch die anderen perfluoralkylhaltigen Verbindungen eine Perfluoralkylkette mit 8 Kohlenstoffatomen aufweisen.

Die neuen galenischen Formulierungen zeigen überraschende Vorteile bei ihrer Verwendung als Kontrastmittel. Galenische Formulierungen, bestehend aus perfluoralkylhaltigen Farbstoffmolekülen und anderen perfluoralkylhaltigen Substanzen, sind in breiter Vielfalt herstellbar. Diese Formulierungen eignen sich zur Fluoreszenzdetektion und visuell erfaßbaren Anfärbung von Lymphknoten nach interstitieller oder intravenöser Applikation.
Gegenüber den bereits bekannten Kontrastmitteln zur Darstellung der Lymphknoten zeigen sie eine verbesserte Verträglichkeit und eine nahezu vollständige Ausscheidung. Weiter ist auch die lokale Verträglichkeit höher als bei den bisher bekannten Kontrastmitteln, und gleichzeitig zeigen die neuen Formulierungen eine höhere Organspezifität. Die Anreicherung in den Lymphknoten ist höher als bei den bekannten Kontrastmitteln für die Lymphographie. Wenn gleichzeitig perfluoralkylhaltige Farbstoffmoleküle und perfluorhaltige Metallkomplexe verwendet werden, ist es möglich, nacheinander verschiedene diagnostische Verfahren anzuwenden. Neben der Nahinfrarotdiagnostik kann auch z.B. Computer- oder Kernspintomographie durchgeführt werden.

Ein weiterer Vorteil besteht darin, daß die Lymphknoten eine charakteristische Färbung annehmen. Dies erlaubt zusätzlich zur NIR-Diagnostik eine intraoperative Fluoreszenzdiagnostik der Lymphknotenmorphologie und Lymphbahnendurchgängigkeit sowie eine fluoreszenzgestützte Entnahme von Biopsien. Besonders wichtig ist dabei die fluoreszenzgestützte Detektion des sogenannten Sentinel-Lymphknotens. Der Sentinel-Lymphknoten ist der erste Lymphknoten, der die Lymphe eines Tumorareals drainiert und somit auch der erste Lymphknoten, der bei einem Metastasenbefall von Lymphknoten betroffen ist.

Die fluoreszenzgestützte Detektion dieser Lymphknoten erfolgt sehr viel einfacher als z.B. die Detektion mit Hilfe von Radiopharmaka, weil der Nachweis von Röntgenstrahlung immer schwieriger ist als eine unmittelbare Detektion der Fluoreszenzstrahlung. Zudem sind die angefärbten Lymphknoten visuell auszumachen, da die Lymphknoten bei entsprechender Dosierung der erfindungsgemäßen Verbindungen auch eine charakteristische Färbung annehmen können.

Die Farbstoffmoleküle werden in Anlehnung an literaturbekannte Methoden hergestellt und dann mit Perfluoralkylderivaten gekoppelt. Bevorzugt sind Farbstoffe aus o. g. Klassen, die Carboxylgruppen oder Isothiocyanatgruppen enthalten. Besonders bevorzugt sind solche Farbstoffe, die Carboxylgruppen enthalten, welche nach Aktivierung mittels gängiger Reagenzien mit Aminogruppen enthaltenden Perfluoralkylderivaten unter Bildung einer Amidgruppe umgesetzt werden.

Literatur zur Synthese von Polymethinfarbstoffen: Bioconjugate Chem. 4, 105-111, 1993; Bioconjugate Chem. 7, 356-62, 1996; Bioconjugate Chem. 8, 751-56, 1997; Cytometry 10, 11-19, 1989 und 11, 418-30, 1990; J. Heterocycl. Chem. 33, 1871-6, 1996; J. Org. Chem. 60, 2391-5, 1995; Dyes and Pigments 17, 19-27, 1991, Dyes and Pigments 21, 227-34, 1993; J. Fluoresc. 3, 153-155, 1993; Anal. Biochem. 217, 197-204, 1994; US 4981977; US 5688966; US 5808044; WO 97/42976; WO 97/42978; WO 98/22146; WO 98/26077; EP 0800831.

Die Herstellung der galenischen Formulierungen erfolgt dadurch, daß die perfluoralkylhaltigen Farbstoffmoleküle (Komponente A) und die anderen perfluoralkylhaltigen Substanzen (Komponente B) eingewogen und in einem geeigneten Lösungsmittel gelöst werden. Ein besonders gut geeignetes Lösungsmittel ist Wasser. Wie oben bereits erwähnt, beträgt der Anteil der perfluoralkylhaltigen Farbstoffmoleküle zwischen 1 und 10 mol% bezogen auf die Gesamtmenge an perfluoralkylhaltigen Substanzen. Die Konzentration der Lösung beträgt vorzugsweise zwischen 0.1 mmol/L und 20 mmol/L bezogen auf den Farbstoff. Dieser Lösung werden dann übliche galenische Zusätze wie z.B. Pufferlösungen und das Ca-Salz des Komplexbildners im Überschuß zugegeben. Bei 10 bis 100°C werden die Lösungen stark gerührt. Alternativ können die Lösungen bei 10 bis 100°C in einem Ultraschallbad behandelt werden. Eine weitere Alternative besteht darin, daß man die Lösungen mit Mikrowellen behandelt.

Bei Stoffen, die sich als Einzelkomponenten nicht in Wasser lösen, erweist es sich als vorteilhaft, einen Lösungsvermittler wie Alkohol (z.B. Methanol oder Ethanol) oder ein anderes mit Wasser mischbares Lösungsmittel zuzusetzen und dieses dann langsam abzudestillieren. Die Destillation kann unter Vakuum erfolgen. Der Rückstand wird anschließend in Wasser gelöst und die Lösung filtriert. Es ist auch möglich, jede Komponente für sich in jeweils einem Lösungsmittel getrennt zu lösen, dann zusammenzufügen und wie oben weiterzuverfahren.

Derart hergestellte Lösungen lassen sich gefriertrocknen. Die gefriergetrockneten Lösungen lassen sich erneut in Wasser lösen und behalten überraschenderweise ihre vorteilhaften Eigenschaften bei. Dies erlaubt eine lange Lagerzeit des Wirkstoffes.

Beim Einsatz als Kontrastmittel für die Darstellung der Lymphknoten werden die wäßrigen Lösungen der perfluoralkylhaltigen Substanzen (in einer Konzentration von zwischen 0,1 mmoL/L und 20 mmol/L bezogen auf den Farbstoff, s.o.) durch interstitielle/intrakutane oder intravenöse Injektion an einer oder mehreren Injektionsstellen appliziert. Das Applikationsvolumen beträgt in Abhängigkeit von der Spezies und der Applikationsform zwischen 0,1 ml und 30 ml, die applizierte Dosis liegt vorzugsweise zwischen 0,1 µmol/kg und 10 µmol/kg Körpergewicht, bezogen auf den Farbstoff.

Nach Injektion der galenischen Formulierung wird Licht aus dem entsprechenden Spektralbereich zur elektronischen Anregung des verwendeten Farbstoffes in das Gewebe eingestrahlt. Das reflektierte Anregungslicht oder die vom Farbstoff emittierte Fluoreszenzstrahlung wird registriert. Bevorzugt sind die Methoden, bei denen das Gewebe großflächig bestrahlt und die Fluoreszenzstrahlung örtlich aufgelöst durch Aufnahme mit einer CCD-Kamera dargestellt wird oder die abzubildenden Gewebeareale mit einem Lichtleiter abgerastert und die erhaltenen Signale rechnerisch in ein synthetisches Bild umgesetzt werden. Dabei kann die Fluoreszenz spektral und/oder phasenselektiv sowie stationär und/oder zeitaufgelöst detektiert und ausgewertet werden. Die erhaltenen Fluoreszenzbilder können simultan mit Weißlichtbildern erzeugt werden und zur Datenauswertung in einer Abbildung übereinander dargestellt werden. Im Fall der intraoperativen Diagnostik kann zusätzlich zur Fluoreszenzdetektion die Anfärbung visuell beobachtet werden.

Die nachfolgenden Beispiele erläutern die Erfindung, ohne sie auf diese beschränken zu wollen.

**<u>Beispiele 1 und 2:</u> Synthese der Bis-Sulfobutyl-Indocyaninfarbstoffe 1,1'-Bis-(4-sulfobutyl)indodicarbocyanin-5-carbonsäure, Natriumsalz (1) und 1,1'-Bis-(4-sulfobutyl)indotricarbocyanin-5-carbonsäure, Natriumsalz (2) zur Kopplung an Perfluoralkylaminoderivate**

Die Synthese erfolgt generell ausgehend von 1-(4-Sulfobutyl)-2,3,3-trimethyl-*3H*-indolenin und 1-(4-Sulfobutyl)-2,3,3-trimethyl-5-carboxy-*3H*-indolenin (Cytometry 10, 11-19, 1989, Talanta 39, 505-510, 1992).

Beispiel 1:

Synthese von 1,1'-Bis-(4-sulfobutyl)indodicarbocyanin-5-carbonsäure, Natriumsalz (**1**)

1,2 g (4,1 mmol) 1-(4-Sulfobutyl)-2,3,3-trimethyl-*3H*-indolenin und 1,0 g ( 3,9 mmol) Malonaldehyd-bis-phenylimin-hydrochlorid werden in 15 ml Essigsäureanhydrid 30 min bei 120 °C gerührt und dann mit einem Wasserbad auf Raumtemp. abgekühlt. Anschließend werden nacheinander 1,4 g (4,2 mmol) 1-(4-Sulfobutyl)-2,3,3-trimethyl-5-carboxy-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die Reaktionslösung abgekühlt und mit 100 ml Ether versetzt. Der ausgefallene Feststoff wird abfiltriert. Es erfolgt eine chromatographische Reinigung an RP-Kieselgel EUROPREP 60-30 C18 (Knauer), 60A, 20-45 μ (Eluens: Wasser/MeOH, Stufengradient von 0 % auf 70 % MeOH). Die produktenthaltenden Fraktionen werden am Rotations-verdampfer vom Methanol befreit und anschließend lyophilisiert, Ausbeute: 1,8 g (66 %), blaues Lyophilisat.

Beispiel 2:

Synthese von 1,1'-Bis-(4-sulfobutyl)indotricarbocyanin-5-carbonsäure, Natriumsalz (**2**)

1,2 g (4,1 mmol) 1-(4-Sulfobutyl)-2,3,3-trimethyl-*3H*-indolenin und 1,1 g ( 3,9 mmol) Glutaconaldehyd-dianilhydrochlorid werden in 15 ml Essigsäureanhydrid 30 min bei 120 °C gerührt und dann mit einem Wasserbad auf Raumtemp. abgekühlt. Anschließend werden 1,4 g (4,2 mmol) 1-(4-Sulfobutyl)-2,3,3-trimethyl-5-carboxy-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Die Aufarbeitung und Reinigung erfolgt wie in Beispiel 1 beschrieben, Ausbeute: 1,8 g (60 %), blaues Lyophilisat.

**Beispiele 3 und 4: Synthese der Mono-Sulfobutyl-Indocyaninfarbstoffe 1-Methyl-1'-(4-sulfobutyl)indodicarbocyanin-5-carbonsäure (3) und 1-Methyl-1'-(4-sulfobutyl)indotricarbocyanin-5-carbonsäure (4) zur Kopplung an Perfluoralkylaminoderivate**

Die Synthese erfolgt generell ausgehend von 1-(4-Sulfobutyl)-2,3,3-trimethyl-*3H*-indolenin und 1,2,3,3-tetramethyl-5-carboxy-*3H*-indolium iodid (Cytometry 10, 11-19, 1989, Talanta 39, 505-510, 1992).

Beispiel 3: Synthese von 1-Methyl-1'-(4-sulfobutyl)indodicarbocyanin-5-carbonsäure (**3**)

1,2 g (4,1 mmol) 1-(4-Sulfobutyl)-2,3,3-trimethyl-*3H*-indolenin und 1,0 g ( 3,9 mmol) Malonaldehyd-bis-phenylimin-hydrochlorid werden in 15 ml Essigsäureanhydrid 30 min bei 120 °C gerührt und dann mit einem Wasserbad auf Raumtemp. abgekühlt. Anschließend werden nacheinander 1,6 g (4,6 mmol) 1,2,3,3-tetramethyl-5-carboxy-3H-indolium iodid, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Der ausgefallene Feststoff wird abfiltriert. Es erfolgt eine chromatographische Reinigung an RP-Kieselgel EUROPREP 60-30 C18 (Knauer), 60A, 20-45 μ (Eluens: Wasser/MeOH, Stufengradient von 30 % auf 90 % MeOH). Die produktenthaltenden Fraktionen werden am Rotationsverdampfer vom Methanol befreit und anschließend lyophilisiert, Ausbeute: 0,9 g (42 %), blaues Lyophilisat.

Beispiel 4: Synthese von 1-Methyl-1'-(4-sulfobutyl)indotricarbocyanin-5-carbonsäure (**4**)

1,2 g (4,1 mmol) 1-(4-Sulfobutyl)-2,3,3-trimethyl-*3H*-indolenin und 1,1 g ( 3,9 mmol) Glutaconaldehyd-dianilhydrochlorid werden in 15 ml Essigsäureanhydrid 30 min bei 120 °C gerührt und dann mit einem Wasserbad auf Raumtemp. abgekühlt. Anschließend werden 1,6 g (4,6 mmol) 1,2,3,3-tetramethyl-5-carboxy-*3H*-indolium iodid, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die Reaktionslösung abgekühlt und mit 100 ml Ether versetzt. Die Aufarbeitung und Reinigung erfolgt wie in Beispiel 3 beschrieben, Ausbeute: 1,4 g (62 %), blaues Lyophilisat.

**Beispiele 5 bis 8: Synthese der perfluoralkylierten Bis-Sulfobutyl-Cyaninfarbstoffe 7 bis 10**

Die Synthese erfolgt aus **1** bzw. **2** durch Aktivierung der Carbonsäure und Umsetzung mit 1H,1H,2H,2H,4H,4H,5H,5H-3-oxa-perfluortridecylamin (**5**) zu **7** und **8**, sowie durch Umsetzung mit N-(2,3-Dihydroxypropyl)-N-(1H,1H,2H,2H,4H,4H,5H,5H-3-oxa-perfluortridecyl)-amin (**6**) zu **9** und **10**. Die Synthese von **5** und **6** ist in DE

199 14 101 beschrieben.

| # | M (g/mol) | |
|---|---|---|
| **7** | 1182,0 | 5-{N-[2-(3,3,4,4,5,5,6,6,7,7,8,8,9,9,10,10,10-Heptadecafluorodecyloxy) ethyl]-aminocarbonyl}-2-{7-[1,3-dihydro-3,3-dimethyl-1-(4-sulfobutyl)-2H-indol-2-ylidene]-1,3-pentadienyl}-3,3-dimethyl-1-(4-sulfobutyl)-3H-indolium, inneres Salz, Natriumsalz |
| **8** | 1208,0 | 5-{N-[2-(3,3,4,4,5,5,6,6,7,7,8,8,9,9,10,10,10-Heptadecafluorodecyloxy) ethyl]-aminocarbonyl}-2-{7-[1,3-dihydro-3,3-dimethyl-1-(4-sulfobutyl)-2H-indol-2-ylidene]-1,3,5-heptatrienyl}-3,3-dimethyl-1-(4-sulfobutyl)-3H-indolium, inneres Salz, Natriumsalz |
| **9** | 1256,1 | 5-{N-[2-(3,3,4,4,5,5,6,6,7,7,8,8,9,9,10,10,10-Heptadecafluorodecyloxy)ethyl]-N-(2,3-dihydroxypropyl)aminocarbonyl}-2-{7-[1,3-dihydro-3,3-dimethyl-1-(4-sulfobutyl)-2H-indol-2-ylidene]-1,3-pentadienyl}-3,3-dimethyl-1-(4-sulfobutyl)-3H-indolium, inneres Salz, Natriumsalz |
| **10** | 1282,1 | 5-{N-[2-(3,3,4,4,5,5,6,6,7,7,8,8,9,9,10,10,10-Heptadecafluorodecyloxy)ethyl]-N-(2,3-dihydroxypropyl)aminocarbonyl}-2-{7-[1,3-dihydro-3,3-dimethyl-1-(4-sulfobutyl)-2H-indol-2-ylidene]-1,3,5-heptatrienyl}-3,3-dimethyl-1-(4-sulfobu-tyl)-3H-indolium, inneres Salz, Natriumsalz |

<u>Beispiel 5 und 6:</u> Synthese von 7 und 8 aus den Farbstoffen 1 und 2

Ein Lösung von 0,5 mmol des Farbstoffes **1** bzw. **2** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,29 g (0,55 mmol) **5** und 0,6 mmol Triethylamin in 5 ml Dimethylformamid zugetropft und das Reaktionsgemisch 2 h bei Raumtemp. gerührt. Nach Zugabe von 50 ml Hexan / 50 ml Ethylacetat wird der ausgefallene Feststoff abfiltriert und chromatographisch an RP-Kieselgel LiChroprep® RP-8 (Merck), 40-63 µ (Eluens: Wasser/MeOH, Stufengradient von 20 % auf 80 % MeOH). gereinigt; Ausbeuten: 0,42 g (71%) **7**, 0,45 g (75%) **8**.

<u>Beispiel 7 und 8:</u> Synthese von 9 und 10 aus den Farbstoffen 1 und 2

Ein Lösung von 0,5 mmol des Farbstoffes **1** bzw. **2** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,37 g (0,65 mmol) **6** in 5 ml Dimethylformamid zugetropft und das Reaktionsgemisch 18 h bei Raumtemp. gerührt. Nach Zugabe von 50 ml Hexan / 50 ml Ethylacetat wird der ausgefallene Feststoff abfiltriert und chromatographisch an RP-Kieselgel LiChroprep® RP-8 (Merck), 40-63 µ (Eluens: Wasser/MeOH, Stufengradient von 20 % auf 80 % MeOH). gereinigt; Ausbeuten: 0,40 g (63%) **9**, 0,43 g (67%) **10**.

**<u>Beispiele 9 bis 12:</u> Synthese der perfluoralkylierten Mono-Sulfobutyl-Cyaninfarbstoffe 11 bis 14**

Die im Beispiel ausgeführte Synthese erfolgt aus **3** bzw. **4** durch Aktivierung der Carbonsäure und Umsetzung mit 1H,1H,2H,2H,4H,4H,5H,5H-3-oxa-perfluortridecylamin (**5**) zu **11** und **12**, sowie durch Unsetzung mit N-(2,3-Dihydroxypropyl)-N-(1H,1H,2H,2H,4H,4H,5H,5H-3-oxa-perfluortridecyl)-amin (**6**) zu **13** und **14**.

| # | M (g/mol) | |
|---|---|---|
| **11** | 1037,9 | 2-[5-(1-Ethyl)-1,3-dihydro-3,3-dimethyl-2H-indol-2-ylidene)-1,3-pentatrienyl]-5-{N-[2-(3,3,4,4,5,5,6,6,7,7,8,8,9,9,10,10,10-heptadecafluorodecyloxy)ethyl]- aminocarbonyl}-3,3-dimethyl-1-(4-sulfobutyl)-3H-indolium, inneres Salz, Natriumsalz |
| **12** | 1063,9 | 2-[5-(1-Ethyl)-1,3-dihydro-3,3-dimethyl-2H-indol-2-ylidene)-1,3,5-heptatrienyl]-5-{N-[2-(3,3,4,4,5,5,6,6,7,7,8,8,9,9,10,10,10-heptadecafluorodecyloxy)-ethyl]- aminocarbonyl}-3,3-dimethyl-1-(4-sulfobutyl)-3H-indolium, inneres Salz, Natriumsalz |

(fortgesetzt)

| # | M (g/mol) | |
|---|---|---|
| **13** | 1112,9 | 2-[5-(1-Ethyl)-1,3-dihydro-3,3-dimethyl-2H-indol-2-ylidene)-1,3-pentatrienyl]-5-{N-[2-(3,3,4,4,5,5,6,6,7,7,8,8,9,9,10,10,10-heptadecafluorodecyloxy)ethyl]- N-(2,3-dihydroxypropyl)aminocarbonyl}-3,3-dimethyl-1-(4-sulfobutyl)-3H-indolium, inneres Salz, Natriumsalz |
| **14** | 1138,9 | 2-[5-(1-Ethyl)-1,3-dihydro-3,3-dimethyl-2H-indol-2-ylidene)-1,3,5-heptatri-enyl]-5-{N-[2-(3,3,4,4,5,5,6,6,7,7,8,8,9,9,10,10,10-heptadecafluorodecyloxy)-ethyl]-N-(2,3-dihydroxypropyl)aminocarbonyl}-3,3-dimethyl-1-(4-sulfobutyl)-3H-indolium, inneres Salz, Natriumsalz |

Beispiel 9 und 10: Synthese von 11 und 12 aus den Farbstoffen 3 und 4

Ein Lösung von 0,5 mmol des Farbstoffes **3** bzw. **4** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,29 g (0,55 mmol) **5** und 0,6 mmol Triethylamin in 5 ml Dimethylformamid zugetropft und das Reaktionsgemisch 2 h bei Raumtemp. gerührt. Nach Zugabe von 100 ml Hexan wird der ausgefallene Feststoff abfiltriert und chromatographisch an RP-Kieselgel LiChroprep® RP-8 (Merck), 40-63 µ (Eluens: Wasser/MeOH, Stufengradient von 30 % auf 90 % MeOH). gereinigt; Ausbeuten: 0,25 g (48%) **11**, 0,35 g (66%) **12**.

Beispiel 11 und 12: Synthese von 13 und 14 aus den Farbstoffen 3 und 4

Ein Lösung von 0,5 mmol des Farbstoffes **1** bzw. **2** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,37 g (0,65 mmol) **6** in 5 ml Dimethylformamid zugetropft und das Reaktionsgemisch 18 h bei Raumtemp. gerührt. Nach Zugabe von 100 ml Hexan wird der ausgefallene Feststoff abfiltriert und chromatographisch an RP-Kieselgel LiChroprep® RP-8 (Merck), 40-63 µ (Eluens: Wasser/MeOH, Stufengradient von 20 % auf 80 % MeOH). gereinigt; Ausbeuten: 0,39 g (70%) **13**, 0,41 g (72%) **14**.

Folgende Verbindungen wurden in analoger Weise durch Umsetzung mit beschriebenen Perluoralkylderivaten erhalten:

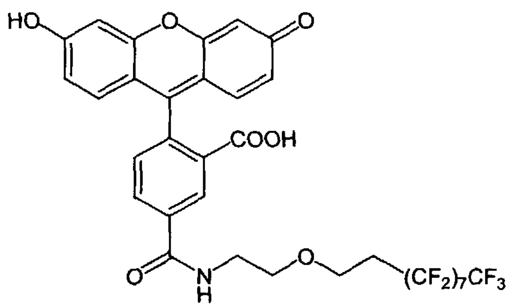

5-Carboxyfluorescein-perfluoralkylamid

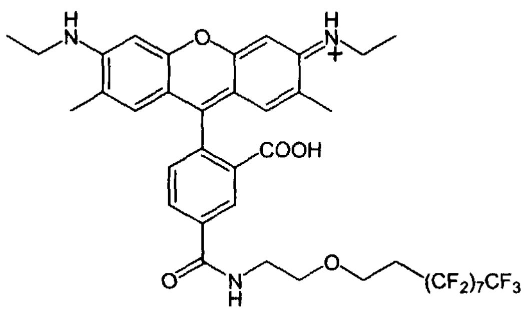

5-Carboxy-Rhodamin6G-perfluoralkylamid

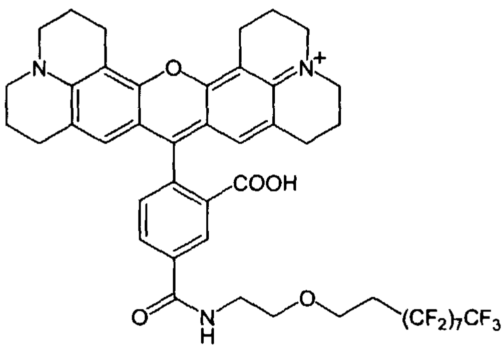

5-Carboxy-X-Rhodamin-perfluoralkylamid

Beispiel 13:

**Darstellung von galenischen Formulierungen aus Gadoliniumkomplexperfluoralkylderivaten am Beispiel von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan mit 1, 2, 5 und 10 mol% der Farbstoffe 7 bis 14**

118 mg (100 µmol) 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan (Herstellung siehe WO 97/26017, Beispiel 33) werden zusammen mit 1 µmol, 2 µmol, 5 µmol bzw. 10 µmol **7** bis **14** in 50 ml Wasser 20 min im Ultraschallbad gelöst. Anschließend wird eine Dialyse zur Abtrennung freien Farbstoffes durchgeführt (Amicon-Zelle, Cut-off 10000, Dialysevolumen 5 × 100 ml). Die Produktlösung wird mit Wasser auf ein Volumen von 5 ml (20 mmol/l an 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan) eingestellt.

Neben 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan wurden folgende Gd-Komplexperfluoralkylderivate zur Herstellung der galenischen Formulierungen verwendet:

- Gadolinium-Komplex von 10-[2-Hydroxy-4-aza-5-oxo-7-oxa-10,10,11,11,12,12,13,13,14,14,15,15,16,16,17,17,17-heptadecafluor-heptadecyl]-1,4,7-tris(carboxymethyl)-1,4,7,10-tetraazacyclododecan (Herstellung siehe DE 196 03 033, Beispiel 2)
- Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan (Herstellung siehe DE 196 03 033, Beispiel 1)
- 1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex

Herstellung:

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3,51 (17 mmol) N,N'-Dicyclohexylcarbodiimid zu und rührt 5 Stunden bei 15 °C. Zur Abtrennung des Harnstoffes wird die Lösung filtriert. Zum Filtrat gibt man 8,63 g (15,88 mmol) 1H, 1H, 2H, 2H, 4H, 4H, 5H, 5H-3-oxa-perfluortridecylamin, Hydrochlorid und 5,06 g (50 mmol) Triethylamin zu und rührt 12 Stunden bei Raumtemperatur. Man gießt die Lösung in 1500 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/ Acetonitril/Wasser).

Ausbeute: 13,86 g (78 % d. Th.) eines farblosen, amorphen Pulvers

Wassergehalt: 9,3 %

| Elementaranalyse (berechnet auf wasserfreie Substanz): | | | | | |
|---|---|---|---|---|---|
| ber.: | C 33,28 | H 3,42 | N 7,51 | F 28,87 | Gd 14,05 |
| gef.: | C 33,12 | H 3,61 | N 7,37 | F 28,69 | Gd 13,89 |

- 1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

<u>Herstellung:</u>

a) 2H, 2H, 4H, 4H, 5H, 5H-3-oxa)-perfluortridecansäure-N-(2,3-dihydroxypropyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 5,47 g (60 mmol) 2,3-Dihydroxypropylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Ethanol= 15:1) chromatographiert.

Ausbeute: 29,70 g (87 % d. Th.) eines farblosen Feststoffes

| Elementaranalyse: | | | | |
|---|---|---|---|---|
| ber.: | C 30,32 | H 2,20 | N 2,36 | F 54,35 |
| gef.: | C 30,12 | H 2,41 | N 2,18 | F 54,15 |

b) N-(2,3-Dihydroxypropyl)-N-(1H, 1H, 2H, 2H, 4H, 4H, 5H, 5H-3-oxa-perfluortridecyl)-amin

30 g (48,8 mmol) der Titelverbindung aus Beispiel a werden in 300 ml Tetrahydrofuran gelöst und 50 ml 10 M Borandimethylsulfid (in Tetrahydrofuran) zugegeben. Man kocht 16 Stunden unter Rückfluß. Es wird auf 0°C abgekühlt und 300 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 60°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/Methanol= 15:1).

Ausbeute: 24,07 g (85 % d. Th.) eines farblosen Feststoffes

| Elementaranalyse: | | | | |
|---|---|---|---|---|
| ber.: | C 31,05 | H 2,61 | N 2,41 | F 55,66 |
| gef.: | C 31,91 | H 2,78 | N 2,33 | F 55,47 |

c) 1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,21 (15,88 mmol) der Titelverbindung aus Beispiel b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).

Ausbeute: 16,09 g (85 % d. Th.) eines farblosen, amorphen Pulvers
Wassergehalt: 6,3 %

| Elementaranalyse (berechnet auf wasserfreie Substanz): | | | | | |
|---|---|---|---|---|---|
| ber.: | C 34,26 | H 3,64 | N 7,05 | F 27,10 | Gd 13,19 |
| gef.: | C 34,12 | H 3,83 | N 6,91 | F 26,88 | Gd 12,93 |

- 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

Herstellung:

a) 2H, 2H, 4H, 4H, 5H, 5H-3-oxa-perfluortridecansäure-N-(5-hydroxy-3-oxa-pentyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 6,25 g (60 mmol) 5-Hydroxy-3-oxa-pentylamin und 6,07 g (60 mmol) Tnethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 15:1) chromatographiert.

Ausbeute: 32,20 g (92 % d. Th.) eines farblosen Feststoffes

| Elementaranalyse: | | | | |
|---|---|---|---|---|
| ber.: | C 31,54 | H 2,65 | N 2,30 | F 53,01 |
| gef.: | C 31,61 | H 2,84 | N 2,14 | F 52,85 |

b) N-(5-Hydroxy-3-oxa-pentyl)-N-(1H, 1H, 2H, 2H, 4H, 4H, 5H, 5H-3-oxa-perfluortridecyl)-amin

30 g (49,24 mmol) der Titelverbindung aus Beispiel a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 10 Stunden bei 50°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).

Ausbeute: 26,09 g (89 % d. Th.) eines farblosen Feststoffes

| Elementaranalyse: | | | | |
|---|---|---|---|---|
| ber.: | C 32,28 | H 3,05 | N 2,35 | F 54,25 |
| gef.: | C 32,12 | H 3,21 | N 2,18 | F 54,09 |

c) 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,45 (15,88 mmol) der Titelverbindung aus Beispiel b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).

Ausbeute: 16,10 g (84 % d. Th.) eines farblosen, amorphen Pulvers
Wassergehalt: 5,7 %

| Elementaranalyse (berechnet auf wasserfreie Substanz): | | | | | |
|---|---|---|---|---|---|
| ber.: | C 34,83 | H 3,84 | N 6,96 | F 26,76 | Gd 13,03 |
| gef.: | C 34,65 | H 3,96 | N 6,84 | F 26,62 | Gd 12,91 |

Beispiel 14:

**Darstellung von galenischen Formulierungen mit Monosaccharidperfluoralkylderivaten am Beispiel von 6-[1-O-α-D-mannopyranosyl)-hexansäure N-(3-oxa-1H,1H,2H,2H,4H,4H,5H,5H-perfluortridecyl)-amid und den Farbstoffen 7 bis 14**

A) Herstellung von 6-[1-O-α-D-mannopyranosyl)-hexansäure N-(3-oxa-1H,1H,2H,2H,4H,4H,5H,5H-perfluortridecyl)-amid

a) 1,2,3,4,6-Penta-O-acetyl-α,β-D-mannopyranose

Auf analoge Weise, wie in der Literatur beschrieben [M.L. Wolfrom und A.Thompson in Methods in Carbohydrate Chemistry (R.L. Whistler, M.L. Wolfrom and J.N. BeMiller, Eds.), Academic Press, New York, Vol.II, 53 , pp. 211-215, (1963)] liefert die Umsetzung von 150 g (832.5 mmol) α,β-D-Mannopyranose mit einem Gemisch aus 1500 ml absolutem Pyridin und 1500 ml Essigsäureanhydrid nach Aufarbeitung 315 g (96,7 %) der oben genannten Titelverbindung als Rohprodukt in Form eines viskosen und farblosen Öls. Durch $^1$H-NMR-spektroskopische Untersuchung der so erhaltenen Titelverbindung konnte das α zu β-Verhältnis beider Anomeren mit 4:1 bestimmt werden. Auf eine Trennung der α,β-Anomeren der oben genannten Titelverbindung kann zur Durchführung der nachfolgenden Reaktionsschritte verzichtet werden.

| Elementaranalyse: | | |
|---|---|---|
| ber.: | C 49,21 | H 5,68 |

(fortgesetzt)

| Elementaranalyse: | | |
|---|---|---|
| gef.: | C 49,12 | H 5,78 |

b) 6-[1-O-α-(2,3,4,6-Tetra-O-acetyl-D-mannopyranosyl)-hexansäureethylester]

Auf analoge Weise, wie in der Literatur für die Synthese von Aryl Glycopyranosiden beschrieben [J. Conchie und G.A. Levvy in Methods in Carbohydrate Chemistry (R.L. Whistler, M.L. Wolfrom and J.N. BeMiller, Eds.) , Academic Press, New York, Vol.II , 90 , pp. 345-347 , ( 1963 )] führt die Umsetzung von 156,2 g ( 400 mmol) der Titelverbindung aus Beispiel Aa) als α, β-Anomerengemisch mit 67 ml ( 400 mmol ) 6-Hydroxy-hexansäureethylester und 60,8 ml (520 mmol) Zinn-IV-chlorid in insgesamt 600 ml 1,2-Dichlorethan nach säulenchromatographischer Aufreinigung (Eluent: Hexan/ Essigsäureethylester 2:1) zur Bildung von 100,05 g (51 % d. Th.) der oben genannten Titelverbindung als farbloses und viskoses Öl. Durch $^1$H-NMR-spektroskopische Untersuchung der so erhaltenen Titelverbindung konnte gezeigt werden, daß es sich bei der oben genannten Titelverbindung ausschließlich um das reine α-Anomere handelt.

| Elementaranalyse: | | |
|---|---|---|
| ber.: | C 52,94 | H 6,77 |
| gef.: | C 52,80 | H 6,78 |

c) 6-[1-O-α-(2,3,4,6-Tetra-O-benzyl-D-mannopyranosyl)-hexansäure

Eine gerührte Suspension von 141,0 g ( 289 mmol ) der Titelverbindung aus Beispiel Ab) in 200 ml Dioxan wird bei Raumtemperatur und unter gleichzeitigem kräftigen Rühren portionsweise mit insgesamt 238,5 g ( 4,26mol ) fein gepulvertem Kaliumhydroxydpulver versetzt . Zur Erhöhung der Rührfähigkeit wird das Reaktionsgemisch mit weiteren 200 ml Dioxan versetzt und die so erhaltene Suspension im Anschluß zur Siedehitze erhitzt und bei dieser Temperatur mit insgesamt 372 ml ( 3,128 mol ) Benzylbromid über einen Zeitraum von zwei Stunden tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,5 Liter Eiswasser langsam eingegossen und die Wasserphase im Anschluß vollständig mit Diethylether extrahiert. Nach dem Waschen der so erhaltenen Etherphase und dem anschließenden Trocknen der selbigen über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180 °C aus dem Reaktionsgemisch abdestilliert. Der so erhaltene, harzig-ölige Rückstand wird an Kieselgel unter Verwendung von Essigsäureethylester/Hexan (1:10) als Eluent gereinigt.

Ausbeute: 172,2, g (91,0 % d. Th.) der oben genannten Titelverbindung in Form eines farblosen und äußerst viskosen Öls

| Elementaranalyse: | | |
|---|---|---|
| ber.: | C 75,68 | H 7,16 |
| gef.: | C 75,79 | H 7,04 |

d) 6-[1-O-α-(2,3,4,6-Tetra-O-benzyl-D-mannopyranosyl)-hexansäure-N-(3-oxa-1H,1H,2H,2H,4H,4H,5H,5H-perfluortridecyl)-amid

In 1200 ml trockenem Tetrahydrofuran werden 100g (134 mmol) der in Beispiel Ac) beschriebenen Säure sowie 13.5 g (134 mmol) Triethylamin gelöst. Nach dem Abkühlen auf -15°C tropft man unter Rühren eine Lösung von 18.45 g (135 mmol) Chlorameisensäureisobutylester in 200 ml trockenem Tetrahydrofuran langsam hinzu, wobei die Innentemperatur -10°C nicht überschreitet. Nach einer Reaktionszeit von 15 Minuten bei -15°C tropft man eine Lösung von 165.5 g (134 mmol) 1-Amino-1H,1H,2H,2H-perfluorodecan und 13.5 g (134 mmol) Triethylamin in 250 ml trockenem Tetrahydrofuran bei -20°C hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 300 ml Essigsäureethylester aufgenommen und zweimal mit je 400 ml gesättigter Natriumhydrogencarbonatlösung sowie einmal mit 500 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (10:5:1) als Eluent gereinigt.

Ausbeute: 143,8 g (86,9 % d. Th.)

| Elementaranalyse: | | | | |
|---|---|---|---|---|
| ber.: | C 57,38 | H 4,98 | N 1,13 | F 26,15 |
| gef.: | C 57,30 | H 5,44 | N 1,01 | F 26,25 |

e) 6-[1-O-α-D-mannopyranosyl)-hexansäure N-(3-oxa-1H,1H,2H,2H,4H,4H,5H,5H-perfluortridecyl)-amid

40,0 g (32,38 mmol) der Titelverbindung aus Beispiel Ad) werden in 750 ml 2-Propanol gelöst und mit 2,0 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit insgesamt 1000ml Diethylether nach dem Absaugen des ausgefallenen Feststoffes 21,52 g (88,0 % d. Th.) der oben genannten Titelverbindung als farbloses und kristallines Pulver mit dem Zersetzungsschmelzpunkt von 88,5 °C.

| Elementaranalyse : | | | | |
|---|---|---|---|---|
| ber.: | C 36,01 | H 5,92 | N 1,75 | F 40,34 |
| gef.: | C 36,07 | H 6,08 | N 1,76 | F 40,66 |

B) Herstellung der Formulierung

100 µmol 6-[1-O-α-D-mannopyranosyl)-hexansäure N-(3-oxa-1H,1H,2H,2H,4H,4H,5H,5H-perfluortridecyl)-amid werden in 0,5 ml Ethanol angelöst, anschließend mit 50 ml Wasser versetzt und 1, 2, 5 und 10 mol% der Farbstoffe 7 bis 14 in fester Form zugegeben. Nach 1 h Behandlung im Ultraschallbad erfolgt eine Dialyse wie in Beipiel 13 beschrieben.

Neben 6-[1-O-α-D-mannopyranosyl)-hexansäure N-(3-oxa-1H,1H,2H,2H,4H,4H,5H,5H-perfluortridecyl)-amid können folgende Monosaccharid-perfluoralkylderivate I — III zur Herstellung von galenischen Formulierungen verwendet werden:

I. 1-O-α-D-[(1-Perfluoroctylsulfonyl-piperazin-4-carbonyl)-pentyl-5]-mannopyranose

a) 1-O-α-D-[(1-Perfluoroctylsulfonylpiperazin-4-carbonyl)-pentyl-5]-2,3,4,6-tetra-O-benzyl-mannopyranose

In 800 ml eines Gemisches aus Terahydrofuran/Acetonitril (Mischungsverhältnis 7:3) werden 74,59 g (100 mmol) der in Beispiel Ac) beschriebenen Säure sowie 10,11 g (100 mmol) Triethylamin gelöst. Anschließend wird bei Raumtemperatur tropfenweise mit 500 ml einer Tetrahydrofuranlösung von 58.0 g (102.0 mmol) 1-Perfluoroctylsulfonylpiperazin; 10,11g (100 mmol) Triethylamin und 16.84 g (110 mmol) 1-Hydroxybenzotriazol versetzt. Die so erhaltene Reaktionslösung wird bei -5 °C mit einer Lösung von 22,7 g (110 mmol ) Dicyclohexylcarbodiimid, gelöst in 100 ml Tetrahydrofüran, versetzt und anschließend bei -5 °C noch für zwei weitere Stunden gerührt. Nach dem Auftauen der Reaktionslösung wird bei Raumtemperatur weitere 12 Stunden gerührt, vom ausgefallenen Dicyclohexylharnstoff abfiltriert und das erhaltene Filtrat im Vakuum bis zur Trockne eingeengt. Der verbleibende Rückstand wird in 600 ml Essigsäureethylester aufgenommen und zweimal mit je 300 ml gesättigter Natriumhydrogencarbonatlösung sowie zweinmal

mit je 300 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Aceton/2-Propanol (16:2:1) als Eluent gereinigt

Ausbeute: 113,01 g (79,8 % d. Th.) eines farblosen und viskosen Öls

| Elementaranalyse: | | | | | |
|---|---|---|---|---|---|
| ber.: | C 58,52 | H 4,27 | N 1,98 | S 2,26 | F 22,80 |
| gef.: | C 58,42 | H 4,41 | N 1,80 | S 2.28 | F 23,02 |

b) 1-O-α-D-[(1-Perfluoroctylsulfonyl-piperazin-4-carbonyl)-pentyl-5]-mannopyranose

50 g (35,30 mmol) der Titelverbindung aus Beispiel Ia) werden in einer Mischung bestehend aus 500 ml 2-Propanol und 50 ml Wasser gelöst und 2 g Palladiumkatalysator (10 % Pd auf Aktivkohle) hinzugegeben. Man hydriert für 12 Stunden bei Raumtemperatur. Es wird vom Katalysator abfiltriert und das Filtrat im Vakuum zur Trockne eingedampft. Der Rückstand wird in 200 ml Methanol gelöst und das Reaktionsprodukt durch Versetzen mit insgesamt 800 ml Diethylether zur Fällung gebracht. Nach dem Absaugen des so erhaltenen Feststoffs wird dieser im Vakuum bei 50°C getrocknet.

Ausbeute: 29,51 g (99 % d. Th.) eines amorphen Feststoffes

| Elementaranalyse: | | | | | |
|---|---|---|---|---|---|
| ber.: | C 34,13 | H 3,46 | N 3,32 | S 3,80 | F 38,23 |
| gef.: | C 34,28 | H 3,81 | N 3,25 | S 3,80 | F 38.01 |

II. 2-Desoxy-2-[acetyl-(2-amino-N-ethyl-N-perfluoroctylsulfonyl)-amino]-1-α,β-D-mannopyranose

a) 2-Acetamido-2-deoxy-1,3,4,6-(tetra-O-benzyl)-α,β-D-glucopyranose

Zu einer gerührten Suspension von 20.16 g ( 700 mmol; 80 % ig in Mineralöl) Natriumhydrid in 150 ml Dimethylsulfoxid gibt man bei Raumtemperatur insgesamt 24,0 g (108,5 mmol) 2-Acetamido-2-deoxy-α,β-D-glucopyranose, gelöst in 500 ml absolutem Dimethylsulfoxid , tropfenweise hinzu. Anschließend laßt man noch 120 Minuten bei Raumtemperatur nachrühren und tropft dann 159,5 g (1,26 mol) Benzylchlorid hinzu. Die so erhaltene Reaktionslösung wird im Anschluß für weitere 12 Stunden bei Raumtemperatur gerührt. Zur Aufarbeitung wird die Reaktionslösung langsam in 1,5 Liter Eiswasser eingegossen und anschließend erschöpfend mit Diethylether extrahiert. Die vereingten Diethyletherphasen werden im Anschluß zweimal mit je 600 ml gesättigter Natriumhydrogencarbonatlösung sowie zweimal mit je 800 ml Wasser gewaschen. Nach dem Trocknen der organischen Phase über Natriumsulfat wird vom Salz abgesaugt und das Lösungsmittel im Vakuum abgezogen. Der verbleibende ölige Rückstand wird an Kieselgel unter Verwendung von Essigsäureethylester/Hexan (1: 5) als Eluent gereinigt.

Ausbeute: 48,68 g ( 73,6 % d. Th.) der oben genannten Titelverbindung in Form eines viskosen und farblosen Öls

| Elementaranalyse: | | | |
|---|---|---|---|
| ber.: | C 70,92 | H 6,45 | N 6,89 |
| gef.: | C 71,43 | H 6,44 | N 7,02 |

b) 1-O-Benzyl-3,4,6-tri-O-benzyl-2-amino-2-deoxy-α,β-D-glucopyranose

30.0 g ( 49,2 mmol) der Titelverbindung aus Beispiel IIa) werden in einer Mischung aus 750 ml Methanol und 215 ml Wasser suspendiert und bei Raumtemperatur mit insgesamt 440 ml (49,2 mmol) einer 0,112 molaren wässrigen Perchlorsäurelösung tropfenweise versetzt. Nach beendeter Zugabe wird die Reaktionslösung noch 10 Minuten bei Raumtemperatur gerührt und die so erhaltene, nun homogene, Reaktionslösung wird im Anschluß im Vakuum bis zur Trockne eingeengt. Durch Versetzen des verbleibenden öligen Rückstandes mit einer Mischung aus gleichen Teilen Hexan und Dichlormethan wird dieser zur Kristallisation gebracht. Das kristalline Reaktionsprodukt wird abgesaugt, mit Hexan gewaschen und im Vakuum bei Raumtemperatur getrocknet.

Ausbeute: 27,08g (86 % d. Th.) an oben genannter Titelverbindung in Form ihres Perchlorates, welches als farblose, kristalline Verbindung vorliegt.
Schmelzpunkt : 180,5 - 181,5 °C

| Elementaranalyse: | | | | |
|---|---|---|---|---|
| ber.: | C 63,68 | H 5,98 | N 2,19 | Cl 5.54 |
| gef.: | C 63,43 | H 6,04 | N 2,02 | Cl 5.71 |

c) 1,3,4,6-Tetra-O-benzyl-2-desoxy-2-[acetyl-(2-amino-N-ethyl-N-perfluoroctylsulfonyl)-amino]-1-α,β-D-mannopyranose

In 350 ml trockenem Tetrahydrofuran werden 20,8 g (35,6 mmol) der 2-[N-Ethyl-N-perfluoroctylsulfonyl)-aminoessigsäure sowie 3,60 g (35,6 mmol) Triethylamin gelöst. Nach dem Abkühlen der Reaktionslösung auf -15°C bis -20°C tropft man bei dieser Temperatur unter Rühren eine Lösung von 4,92 g (35,6 mmol) Chlorameisensäureisobutylester in 75 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man anschließend eine Lösung von 22,78 g (35,6 mmol) des Perchlorats (Titelverbindung aus Beispiel IIb) und 3,60g ( 35,6 mmol) Triethylamin, in 100 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 100 ml gesättigter Natriumhydrogencarbonatlösung sowie einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester/Hexan (1:5) als Eluent gereinigt.

Ausbeute: 33,3 g ( 84,6 % d. Th.) der oben genannten Titelverbindung als farbloses und stark viskoses Öl

| Elementaranalyse: | | | | | |
|---|---|---|---|---|---|
| ber.: | C 49,92 | H 3,92 | N 2,53 | F 29,18 | S 2,90 |
| gef.: | C 49,99 | H 4,11 | N 2,69 | F 29,22 | S 3,01 |

d) 2-Desoxy-2-[acetyl-(2-amino-N-ethyl-N-perfluoroctylsulfonyl)-amino]-1-α,β-D-mannopyranose

20,0 g ( 18,06 mmol) der Titelverbindung aus Beispiel IIc) werden in 250 ml 2-Propanol gelöst und mit 1,5 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit 750 ml einer Mischung aus gleichen Teilen Diethylether und Essigsäureethylester nach dem Absaugen des ausgefallenen Feststoffes 12,65g (93,8 % d. Th.) der oben genannten Titelverbindung als farbloses und kristallines Pulver. Die oben genannte Titelverbindung liegt als α/β-Anomerengemisch vor, wobei das Verhältnis bezüglich der beiden möglichen Anomeren durch $^1$H-NMR-spektroskopische Untersuchungen zu ca. 1:1,2 bestimmt wurde. Demnach handelt es sich bei der Titelverbindung um ein fast annähernd gleichverteiltes α/β-Anomerengemisch .

Schmelzpunkt: 132,5 - 133 °C.

| Elementaranalyse: | | | | | |
|---|---|---|---|---|---|
| ber.: | C 28,97 | H 2,57 | N 3,75 | F 43,27 | S 4,30 |
| gef.: | C 29,09 | H 2,56 | N 3,84 | F 43,36 | S 4,42 |

III. 1-O-β-D-[6-Hexansäure-N-(3-oxa-1H,1H,2H,2H,4H,4H,5H,5H-perfluortridecyl)-amid]-glucopyranose

a) 1,2,3,4,6-Penta-O-acetyl-α-D-glucopyranose

Auf analoge Weise, wie zur Synthese der Titelverbindung Aa) beschrieben, liefert die Umsetzung von 100 g (555,0 mmol) α -D-Glucopyranose mit einem Gemisch aus 1000 ml absolutem Pyridin und 1000 ml Essigsäureanhydrid nach Aufarbeitung und Umkristallisation aus 95 %igem wässrigem Ethanol 190,6 g (88,0 %) der oben genannten Titelverbindung als farblose und kristalline Verbindung. Durch $^1$H-NMR-spektroskopische Untersuchung der so erhaltenen Titelverbindung konnte das α zu β-Verhältnis von beiden möglichen Anomeren mit ≥ 98:2 bestimmt werden. Demnach handelt es sich bei der Titelverbindung um das ausschließlich α-konfigurierte Anomere.

Schmelzpunkt : 110,5 °C

| Elementaranalyse: | | |
|---|---|---|
| ber.: | C 49,21 | H 5,68 |
| gef.: | C 49,24 | H 5,68 |

b) 5-(Ethoxycarbonyl)pentyl -2,3,4,6-tetra-O-acetyl-α-D-mannopyranosid

Auf analoge Weise, wie bei der Synthese der Titelverbindung aus Beispiel Ab) beschrieben, liefert die Umsetzung von 130,0 g ( 332,8 mmol) der Titelverbindung aus Beispiel IIIa) mit 55.8 ml ( 332,8 mmol ) 6-Hydroxy-hexansäureethylester und 50,6 ml (520 mmol) Zinn-IV-chlorid in 500 ml 1,2-Dichlorethan nach säulenchromatographischer Aufreinigung (Eluent : Hexan/Essigsäureethylester 2:1) 101,85 g (62,4 % d. Th.) der oben genannten Titelverbindung als farbloses und viskoses Öl. Nach $^1$H-NMR-spektroskopischer Untersuchung der Titelverbindung konnte anhand der Größe der Kopplungskonstanten von $J_{1,2}$ =8,8 Hz eindeutig auf das Vorliegen der β-Konfiguration am anomeren Zentrum geschlossen werden, welche zudem die einzig vorliegende Konfiguration am Anomeriezentrum darstellt. Somit konnte die oben genannte Titelverbindung nur in Form des β-konfigurierten Anomeren dargestellt werden.

| Elementaranalyse: | | |
|---|---|---|
| ber.: | C 52,94 | H 6,77 |
| gef.: | C 52,77 | H 6,70 |

c) 5-(Carboxy)pentyl -2,3,4,6-tetra-O-benzyl-α-D-mannopyranosid

Eine gerührte Suspension von 100,0 g (204,96 mmol) der Titelverbindung aus Beispiel IIIb) in 150 ml Dioxan wird bei Raumtemperatur und unter gleichzeitigem, kräftigen Rühren portionsweise mit insgesamt 169,14 g ( 3,02 mol ) fein gepulvertem Kaliumhydroxydpulver versetzt. Zur Erhöhung der Rührfähigkeit wird das Reaktionsgemisch mit weiteren 150 ml Dioxan versetzt und die so erhaltene Suspension im Anschluß zur Siedehitze erhitzt und bei dieser Temperatur mit insgesamt 264 ml (2,218 mol) Benzylbromid über einen Zeitraum von zwei Stunden tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,0 Liter Eiswasser langsam eingegossen und die Wasserphase im Anschluß vollständig mit Diethylether extrahiert. Nach dem Waschen der so erhaltenen Etherphase und dem anschließenden Trocknen der organischen Phase über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180°C aus dem Reaktionsgemisch abdestilliert. Der so erhaltene , verbleibende ölige Rückstand wird an Kieselgel unter Verwendung von Essigsäureethylester/Hexan (1:10) als Eluent gereinigt.

Ausbeute: 128,8 g (84,3 % d. Th.) der oben genannten Titelverbindung in Form eines farblosen und äußerst viskosen Öls

| Elementaranalyse: | | |
|---|---|---|
| ber.: | C 75,68 | H 7,16 |
| gef.: | C 75,66 | H 7,23 |

d) 2,3,4,6-Tetra-O-benzyl-1-O-β-D-[6-hexansäure-N-(3-oxa-1H,1H,2H,2H,4H,4H, 5H,5H-perfluortridecyl)-amid]-glucopyranose

In 825 ml trockenem Tetrahydrofuran werden 68,5 g ( 91,79 mmol) der in Beispiel IIIc) beschriebenen Säure sowie 9,25 g (91,79 mmol) Triethylamin gelöst. Nach dem Abkühlen der Reaktionslösung auf -15°C bis -20°C tropft man bei dieser Temperatur unter Rühren eine Lösung von 12,64 g (92,5 mmol) Chlorameisensäureisobutylester in 150 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man anschließend eine Lösung von 46.40 g (91,79 mmol) 1H, 1H, 2H,2H-heptadecafluoro-1-(2-aminoethyoxy)-decan und 9,25 g (91,79 mmol) Triethylamin , als Lösung in 200 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 300 ml gesättigter Natrium-hydrogencarbonatlösung sowie einmal mit 400 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (10:5:1) als Eluent gereinigt.

Ausbeute: 104,7 g (92,4% d. Th.) der o. g. Titelverbindung als farbloses und stark viskoses Öl

| Elementaranalyse: | | | | |
|---|---|---|---|---|
| ber.: | C 57,38 | H 4,98 | N 1,13 | F 26,15 |
| gef.: | C 57,27 | H 5,09 | N 1,11 | F 26,08 |

e) 1-O-β-D-[6-Hexansäure-N-(3-oxa-1H,1H,2H,2H,4H,4H,5H,5H-perfluortridecyl)-amid]-glucopyranose

40,0 g (32,38 mmol) der Titelverbindung aus Beispiel IIId) werden in 750 ml 2-Propanol gelöst und mit 2,0 g Palladium-Katalysator (10 % Pd/C) versetzt. Die Reaktionslösung wird für 12 Stunden bei 22°C und 1 Atmosphäre Wasserstoffdruck hydriert. Anschließend filtriert man vom Katalysator ab und engt das Filtrat zur Trockne ein. Der verbleibende Rückstand wird in 300 ml Dimethylsulfoxid aufgenommen und aus der so erhaltenen Produktlösung erhält man durch Versetzen mit insgesamt 1000ml Diethylether und nachfolgendem Absaugen des ausgefallenen Feststoffes 22,05 g (90,2 % d. Th.) der Titelverbindung als farbloses und kristallines Pulver mit einem Zersetzungsschmelzpunkt von 122-124 °C.

| Elementaranalyse : | | | | |
|---|---|---|---|---|
| ber.: | C 36,01 | H 5,92 | N 1,75 | F 40,34 |
| gef.: | C 36,07 | H 6,08 | N 1,76 | F 40,66 |

Beispiel 15:

**Darstellung von galenischen Formulierungen aus drei Komponenten am Beispiel von 6-[1-O-α-D-mannopyranosyl)-hexansäure N-(3-oxa-1H,1H,2H,2H,4H,4H,5H,5H-perfluortridecyl)-amid, 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan und den Farbstoffen 7 bis 14**

Die Darstellung wurde analog der allgemeinen Vorschrift von Beispiel 14 unter Verwendung verschiedener Anteile 6-[1-O-α-D-mannopyranosyl)-hexansäure N-(3-oxa-1H,1H,2H,2H,4H,4H,5H,5H-perfluortridecyl)-amid und 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan, sowie von jeweils 5 mol% der Farbstoffe 7 bis 14 durchgeführt. Dabei wurde 6-[1-O-α-D-mannopyranosyl)-hexansäure N-(3-oxa-1H,1H,2H,2H,4H,4H,5H,5H-perfluortridecyl)-amid in Ethanol angelöst, mit einer Lösung von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan in Wasser versetzt und nach Zugabe von Farbstoff das Gemisch im Ultraschallbad behandelt und wie in Beispiel 13 verfahren.

Beispiel 16:

**Photophysikalische Charakterisierung der Formulierungen**

Die Bestimmung der Absorptionsmaxima und Extinktionskoeffizienten erfolgte mittels eines Lambda 2-Spektrometers (Fa. Perkin-Elmer). Die Fluoreszenzeigenschaften wurden am SPEX-Fluorolog (Fa. Instruments S.A., Photomultiplier Hamamatsu PM928, Anregung 350W Xenon-Lampe) ermittelt. Die Bestimmung der Fluoreszenzquantenausbeute erfolgte relativ zu Indocyaningrün ($\phi$ = 13% in DMSO) durch Anregung bei 585 nm (Farbstoffe mit p=2) bzw. 685 nm (Farbstoffe mit p = 3), jeweils von Lösungen der Konzentration 2µM an Farbstoff, und Korrektur mit der spektralen Empfindlichkeit von Lampe und Detektor.

Absorptions- und Fluoreszenzeigenschaften von Formulierungen mit 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan in Abhängigkeit des Farbstoffanteils am Beispiel von Farbstoff **7** und **8**

| Farbstoff | Mol% Farbstoff | Absorptionsmax. (nm) | Extinktionskoeff. (L $mol^{-1}$ $cm^{-1}$) | Fluoreszenzmax. (nm) | Fluoreszenzquantenausbeute |
|---|---|---|---|---|---|
| **7** | 1 | 652 | 132000 | 681 | 0,17 |
| **7** | 2 | 651 | 130200 | 683 | 0,18 |
| **7** | 5 | 651 | 123700 | 685 | 0,12 |
| **7** | 10 | 649 | 110900 | 685 | 0,04 |
| **8** | 1 | 755 | 150500 | 784 | 0,23 |
| **8** | 2 | 755 | 148800 | 785 | 0,23 |
| **8** | 5 | 753 | 120800 | 788 | 0,14 |
| **8** | 10 | 751 | 108500 | 790 | 0,03 |

Absorptions- und Fluoreszenzeigenschaften der galenischen Formulierung mit 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan und den Farbstoffen **7-14** in einem Anteil von 5 mol%

| Farbstoff | Mol% Farbstoff | Absorptionsmax. (nm) | Extinktionskoeff. (L $mol^{-1}$ $cm^{-1}$) | Fluoreszenzmax. (nm) | Fluoreszenzquantenausbeute |
|---|---|---|---|---|---|
| **7** | 5 | 651 | 123700 | 685 | 0,12 |
| **8** | 5 | 753 | 120800 | 788 | 0,14 |
| **9** | 5 | 653 | 145000 | 683 | 0,14 |
| **10** | 5 | 754 | 118800 | 788 | 0,13 |
| **11** | 5 | 650 | 150700 | 684 | 0,16 |
| **12** | 5 | 753 | 124100 | 789 | 0,09 |
| **13** | 5 | 653 | 139400 | 682 | 0,12 |
| **14** | 5 | 754 | 120900 | 789 | 0,11 |

Absorptions- und Fluoreszenzeigenschaften der galenischen Formulierungen mit 6-[1-O-α-D-mannopyranosyl)-hexansäure N-(3-oxa-1H,1H,2H,2H,4H,4H,5H,5H-perfluortridecyl)-amid, 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan und dem Farbstoff **8** in einem Anteil von 5 mol%

| Farbstof f | Mol% Farbstoff | Mol% Gd-Komplex | Mol% Monosaccharid | Absorptionsmaximum (nm) | Extinktionskoeffizient (L $mol^{-1}$ $cm^{-1}$) | Fluoreszenzmaximum (nm) | Fluoreszenzquantenausbeute |
|---|---|---|---|---|---|---|---|
| **8** | 5 | 0 | 95 | 760 | 90500 | 800 | 0.16 |
| **8** | 5 | 10 | 85 | 760 | 102700 | 800 | 0.11 |
| **8** | 5 | 30 | 65 | 760 | 107900 | 800 | 0.14 |
| **8** | 5 | 50 | 45 | 756 | 108700 | 798 | 0.26 |
| **8** | 5 | 70 | 25 | 756 | 111100 | 794 | 0.18 |
| **8** | 5 | 90 | 5 | 755 | 112500 | 793 | 0.13 |

Beispiel 17:

**Herstellung von Lyophilisaten und Resuspendierung**

Die gemäß der Vorschriften von Beispiel 13 bis 15 hergestellten galenischen Formulierungen werden nach gängigen Methoden gefriergetrocknet. Die blau gefärbten Lyophilisate werden 5 Tage bei Raumtemperatur gelagert, anschließend in 5-10 ml Wasser durch Schütteln resuspendiert und wie oben beschrieben einer Dialyse unterworfen. Der Farbstoffgehalt nach Dialyse wurde photometrisch bestimmt betrug in allen Fällen 96 — 99 % bezogen auf den Gehalt vor Gefriertrocknung und Resuspendierung.

**Beispiel 18: Interstitielle Nahinfrarot-Lymphographie an Meerschweinchen mit einer Formulierung mit 5 mol% Farbstoff 8 / 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan.**

Die bildgebenden Eigenschaften der erfindungsgemäßen Formulierungen wurden in vivo in Meerschweinchen untersucht. Dazu wurde eine Formulierung interstitiell/intrakutan appliziert und die Anreicherung in regionalen

Lymphknoten in einem Zeitraum von 0 bis 120 Minuten beobachtet. Die Fluoreszenz der Substanzen wurde durch Bestrahlung der Tiere mit Nahinfrarot-Licht der Wellenlänge 740 nm, das mit einem Nd:YAG Laser erzeugt wurde, angeregt. Die Fluoreszenzstrahlung wurde bei einer Wellenlänge von > 800 nm durch eine intensivierte CCD-Kamera detektiert und die Fluoreszenzbilder digital gespeichert.

Fig. 1 zeigt Nahinfrarot-Fluoreszenzbilder eines Meerschweinchens nach interstitieller/intrakutaner Applikation von Formulierungen mit 5 mol% Farbstoff **8** / 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan. Es wurden 0,1 ml einer Stammlösung mit 1 mmol/L **8** in eine Hautfalte zwischen den Zehen der rechten hinteren Extremität injiziert, die Endkonzentration betrug 0,3 µmol/kg **8**.

**A**: Ventrale Ansicht, 120 min nach Applikation, der rechte hintere Fuß des Tieres wurde bei der Aufnahme abgedeckt, da die Injektionsstelle ein starkes Fluoreszenzsignal aufwies.
**B**: Rechtslaterale Ansicht, 117 min nach Applikation, das rechte hintere Bein des Tieres wurde bei der Aufnahme abgedeckt, da die Injektionsstelle ein starkes Fluoreszenzsignal aufwies.

**Beispiel 19: Färbung regionaler Lymphknoten nach interstitieller Applikation einer Formulierung mit 5 mol% Farbstoff 8 / 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan.**

Nach Abschluß der interstitiellen Nahinfrarot-Lymphographie wurden die regionalen Lymphknoten im Bereich der Applikationsstelle präpariert. Es wurde beobachtet, daß diese Lymphknoten infolge der Akkumulation der erfindungsgemäßen Substanz eine grüne Färbung aufwiesen. Diese Färbung kann für die intraoperative Diagnostik zur die Identifizierung der ableitenden Lymphknoten eines bestimmten Gewebebereiches und zur Abgrenzung der Lymphknoten vom umliegenden Gewebe genutzt werden.

Fig. 2 zeigt zwei inguinale Lymphknoten von Meerschweinchen nach interstitieller/intrakutaner Applikation einer Formulierung mit 5 mol % Farbstoff **8** / 95 mol % 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan (**A, B**) sowie einen Lymphknoten eines unbehandelten Meerschweinchens ex vivo (**C**). Eine Formulierung mit 5 mol % des Farbstoffs **8** führte nach interstitieller Applikation zu einer deutlich sichtbaren Grünfärbung der Lymphknoten.

**Beispiel 20: Lokalisation der Formulierung mit 5mol% Farbstoff 8 / 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan in Lymphknoten.**

Die Lokalisation der erfindungsgemäßen Formulierungen in Lymphknoten wurde fluorezenzmikroskopisch an Gefrierschnitten der Lymphknoten untersucht. Dazu wurden die Lymphknoten nach der Lymphographie präpariert und bei —80 °C tiefgefroren. An einem Gefriermikrotom wurden Schnitte von 5 µm Dicke hergestellt. Die Auswertung erfolgte an einem Zeiss Axiovert 135-Fluoreszenzmikroskop, das mit einem Cy 7-(Anregungsfilter HQ 710/70nm, Emissionsfilter 810/90nm, Strahlteiler 750nm LP) ausgestattet war. Von allen Präparaten wurden Weißlicht- und Fluoreszenzbilder mit einer CCD-Kamera (Princeton Instruments RTE/CCD-576) aufgenommen und digital gespeichert.

Fig. 3 zeigt eine Hellfeld- (1a) und eine Nahinfrarot-Fluoreszenzaufnahme (1b) eines Gefrierschnittes aus einem poplietalen Meerschweinchen-Lymphknoten vier Stunden nach interstitieller Applikation einer Formulierung mit 5mol% Farbstoff **8** / 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan. Zum Vergleich sind Aufnahmen eines Kryoschnittes aus einem poplietalen Lymphknoten eines unbehandelten Meerschweinchens im Hellfeld (2a) und in Nahinfrarot-Fluoreszenz (2b) gezeigt. Lymphknoten aus Meerschweinchen die eine interstitielle/intrakutane Injektion der Formulierung erhalten hatten, zeigten eine ausgeprägte Fluoreszenz im nahinfraroten Wellenlängenbereich, wohingegen die Lymphknoten unbehandelter Tiere keine Fluoreszenz aufwiesen.

Aufnahmeparameter:
Hellfeld: Objektiv 2,5x, Belichtung 0,02 sec, keine Akkumulation, Tubusvergrößerung 0,6.
NIR: Objektiv 2,5x, Belichtung 1 sec, Akkumulation 5x, Tubusvergrößerung 0,6, Cy7-Filtersatz.

**Beispiel 21: Fluoreszenzfärbung von Lymphknoten nach intravenöser Applikation einer Formulierung mit 5 mol% Farbstoff 8 und 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan.**

Von der Substanz wurden 0,2 ml einer Lösung, die 0,5 mmol/L Farbstoff **8** und 9,5 mmol/L 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan enthielt, intravenös in eine Schwanzvene der Ratte appliziert. Fünf Stunden nach Applikation wurden die Lymphknoten präpariert und ein Fluoreszenzbild aufgenommen. Die Fluoreszenz der Substanzen wurde durch Bestrahlung der Lymphknoten mit Nahinfrarot-Licht der Wellenlänge 740 nm, das mit einem Nd:YAG Laser erzeugt wurde, angeregt. Die Fluoreszenzstrahlung wurde bei einer Wellenlänge von > 800 nm durch eine intensivierte CCD-Kamera detektiert und die Fluoreszenzbilder digital gespeichert.

Fig 4 zeigt ein Fluoreszenzbild von Lymphknoten 5 Stunden nach intravenöser Applikation einer Formulierung mit 5 mol% Farbstoff **8** und 95 mol% 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan. Gezeigt sind die axillaren (a, a‘), inguinalen (b, b‘) und poplitealen (c, c‘) Lymphknoten des Tieres. Alle Lymphknoten zeigten ein deutliches Fluoreszenzsignal.

**Beispiel 22: Darstellung von Lymphknoten in situ nach interstitieller Applikation einer Formulierung mit 5 mol% Farbstoff 8 und 95 mol% 6-[1-O-α-D-mannopyranosyl)-hexansäure N-(3-oxa-1H,1H,2H,2H,4H,4H,5H,5H-perfluortridecyl)-amid in Ratten.**

Es wurden 0,1 ml einer Formulierung, die 1 mmol/L Farbstoff **8** und 19 mmol/L 6-[1-O-α-D-mannopyranosyl)-hexansäure N-(3-oxa-1H,1H,2H,2H,4H,4H,5H,5H-perfluortridecyl)-amid enthielt, interstitiell in eine Hautfalte zwischen den Zehen der rechten hinteren Extremität appliziert. Fünf Stunden nach Applikation wurde das Versuchstier getötet und ventral eröffnet. Anhand des Fluoreszenzbildes lassen sich Lymphknoten in situ lokalisieren und Lymphbahnen darstellen. Die Identifizierung der Lymphknoten in situ ist auch visuell, durch eine grüne Färbung, die nach interstitieller Applikation der Formulierung hervorgerufen wird, möglich. Die Fluoreszenz der Substanzen wurde durch Bestrahlung des ventral eröffneten Tieres mit Nahinfrarot-Licht der Wellenlänge 740 nm, das mit einem Nd:YAG Laser erzeugt wurde, angeregt. Die Fluoreszenzstrahlung wurde bei einer Wellenlänge von > 800 nm durch eine intensivierte CCD-Kamera detektiert und die Fluoreszenzbilder digital gespeichert.

Fig 5 zeigt ein Fluoreszenzbild einer ventral eröffneten Ratte 5 Stunden nach interstitieller Applikation einer Formulierung mit 5mol% Farbstoff **8** und 95 mol% Mannose in eine Hautfalte zwischen den Zehen der rechten hinteren Extremität. Der axillare und der inguinale Lymphknoten sowie eine verbindende Lymphbahn sind aufgrund eines Fluoreszenzsignals dargestellt.

**Beispiel 23: Fluoreszenzfärbung von Lymphknoten nach interstitieller Applikation einer Formulierung mit 5 mol% Farbstoff 8 und 95 mol% 6-[1-O-α-D-mannopyranosyl)-hexansäure N-(3-oxa-1H,1H,2H,2H,4H,4H,5H,5H-perfluortridecyl)-amid.**

Von der Substanz wurden 0,1 ml einer Lösung, die 1 mmol/L Farbstoff **8** und 19 mmol/L 6-[1-O-α-D-mannopyranosyl)-hexansäure N-(3-oxa-1H,1H,2H,2H,4H,4H,5H,5H-perfluortridecyl)-amid enthielt, interstitiell in eine Hautfalte zwischen den Zehen der rechten hinteren Extremität des Tieres appliziert. Fünf Stunden nach Applikation wurden die Lymphknoten der rechten und linken Körperseite präpariert und ein Fluoreszenzbild aufgenommen. Die Fluoreszenz der Substanzen wurde durch Bestrahlung der Lymphknoten mit Nahinfrarot-Licht der Wellenlänge 740 nm, das mit einem Nd:YAG Laser erzeugt wurde, angeregt. Die Fluoreszenzstrahlung wurde bei einer Wellenlänge von > 800 nm durch eine intensivierte CCD-Kamera detektiert und die Fluoreszenzbilder digital gespeichert.

Fig 6 zeigt ein Fluoreszenzbild von Lymphknoten 5 Stunden nach interstitieller Applikation einer Formulierung mit 5 mol% Farbstoff **8** und 95 mol% 6-[1-O-α-D-mannopyranosyl)-hexansäure N-(3-oxa-1H,1H,2H,2H,4H,4H,5H,5H-perfluortridecyl)-amid. Gezeigt sind die mandibularen (m, m‘), axillaren (a, a‘), mesenterialen (mes), iliacalen (il, il‘), inguinalen (in, in‘), glutealen (g, g') und poplitealen (p, p‘) Lymphknoten des Tieres. Die meisten Lymphknoten der rechten (applizierten) Körperseite zeigten ein Fluoreszenzsignal wohingegen kein Lymphknoten der linken Körperseite (jeweils mit **‘** gekennzeichnet) ein Fluoreszenzsignal aufwies. Die fluoreszenzmarkierten Lymphknoten waren aufgrund der Substanzakkumulation grünlich gefärbt.

## Patentansprüche

1. Galenische Formulierung, dadurch gekennzeichnet, daß sie perfluoralkylhaltige Farbstoffmoleküle und andere perfluoralkylhaltige Substanzen enthält.

2. Formulierung gemäß Anspruch 1, dadurch gekennzeichnet, daß die anderen perfluoralkylhaltigen Substanzen perfluoralkylhaltige Metallkomplexe sind.

3. Formulierung gemäß Anspruch 2, dadurch gekennzeichnet, daß die Formulierungen außer den perfluoralkylhaltigen Farbstoffmolekülen und den perfluoralkylhaltigen Metallkomplexen weitere perfluoralkylhaltige Substanzen

enthalten.

**4.** Formulierung gemäß Anspruch 1, dadurch gekennzeichnet, daß die perfluoralkylhaltigen Farbstoffmoleküle und die anderen perfluoralkylhaltigen Verbindungen in einem Lösungsmittel gelöst vorliegen.

**5.** Formulierung gemäß Anspruch 4, dadurch gekennzeichnet, daß das Lösungsmittel Wasser ist.

**6.** Formulierung gemäß Anspruch 4, dadurch gekennzeichnet, daß der Anteil der perfluoralkylhaltigen Farbstoffmoleküle zwischen 1 und 10 mol% bezogen auf die Gesamtmenge an perfluoralkylhaltigen Substanzen beträgt.

**7.** Formulierung gemäß einem der Ansprüche 1 bis 3, dadurch gekennzeichnet, daß die perfluoralkylhaltigen Farbstoffmoleküle in einem Spektralbereich zwischen 400 und 900 nm absorbieren und fluoreszieren.

**8.** Formulierung gemäß Anspruch 1, dadurch gekennzeichnet, daß die perfluoralkylhaltigen Farbstoffmoleküle Substanzen der allgemeinen Formel I sind:

$$R_f — L — A \qquad (I)$$

worin $R_f$ einen geradkettigen oder verzweigten Perfluoralkylrest mit 4 bis 30 Kohlenstoffatomen darstellt, L für einen Linker und A für ein Farbstoffmolekül steht.

**9.** Formulierung gemäß Anspruch 8, wobei der Linker L eine direkte Bindung oder eine geradkettige oder verzweigte Kohlenstoffkette mit bis zu 20 Kohlenstoffatomen darstellt, welche mit ein oder mehreren —OH, -COOH, $-SO_3$-Gruppen substituiert sein kann und/oder gegebenenfalls durch eine oder mehrere —O-, -S-, -CO-, -CONH-, -NHCO-, -CONR-, -NRCO-, $-SO_2-$, -NH-, -NR-Gruppen oder ein Piperazin unterbrochen ist, wobei R für einen $C_1$- bis $C_{10}$-Alkylrest steht, welcher gegebenenfalls mit einer oder mehreren OH-Gruppen substituiert und/oder durch ein oder mehrere Sauerstoffatome unterbrochen ist.

**10.** Formulierung gemäß Anspruch 8, dadurch gekennzeichnet, daß das Farbstoffmolekül A ein Farbstoff aus der Klasse der Polymethinfarbstoffe, Xanthinfarbstoffe oder der heteroaromatischen, kationischen Farbstoffe ist.

**11.** Formulierung gemäß Anspruch 8, dadurch gekennzeichnet, daß das Farbstoffmolekül A ein Cyaninfarbstoff, Sqariliumfarbstoff, Croconiumfarbstoff, Oxonolfarbstoff, Merocyaninfarbstoff, Kryptocyaninfarbstoff, Fluorescein, Rhodamin, Oxazin, Phenoxazin, Thiazin oder Phenothiazin ist.

**12.** Formulierung gemäß Anspruch 8, dadurch gekennzeichnet, daß das Farbstoffmolekül A ein Molekül gemäß Formel II ist:

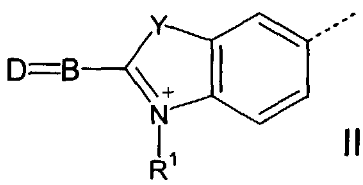

II

worin

D für ein Fragment entsprechend den allgemeinen Formeln III bis VI steht, wobei die mit einem Stern gekennzeichnete Position die Verknüpfung mit B bedeutet:

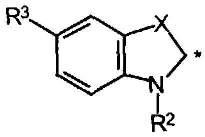
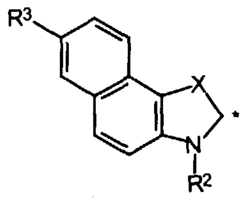
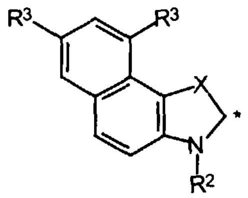
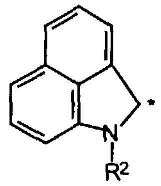

III IV V VI

und worin B für ein Fragment entsprechend den allgemeinen Formeln VII bis XII steht:

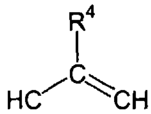
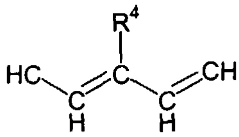
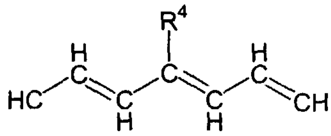

VII VIII IX

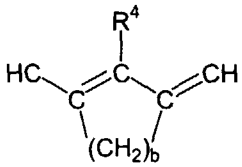
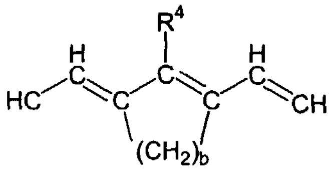
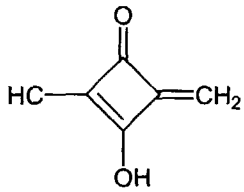

X XI XII

wobei $R^1$ und $R^2$ eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$-$C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

$R^3$ für einen Rest -$COOE^1$, -$CONE^1E^2$, -$NHCOE^1$, -$NHCONHE^1$, -$NE^1E^2$, - $OE^1$, -$OSO_3E^1$, -$SO_3E^1$, -$SO_2NHE^1$, -$E^1$ steht,

wobei $E^1$ und $E^2$ unabhängig voneinander ein Wasserstoffatom, eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$- $C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

und wobei $R^4$ für ein Wasserstoffatom, ein Fluor-, Chlor-, Brom-, Iodatom oder eine verzweigte oder geradkettige $C_1$-$C_{10}$-Alkylkette steht,

b eine Zahl 2 oder 3 bedeutet,

sowie X und Y unabhängig voneinander O, S, -CH=CH- oder $C(CH_3)_2$ bedeuten.

**13.** Formulierung gemäß Anspruch 2, dadurch gekennzeichnet, daß die perfluoralkylhaltigen Metallkomplexe ausgewählt sind aus der folgenden Gruppe von Metallkomplexen:
der Gadoliniumkomplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan,
der Gadoliniumkomplex von 10-[2-Hydroxy-4-aza-5-oxo-7-oxa-10,10,11,11,12,12,13,13,14,14,15,15,16,16,17,17,17-heptadecafluorheptadecyl]-1,4,7-tris(carboxymethyl)-1,4,7,10-tetraazacyclododecan,
1,4,7-Tris{1,4,7-tris(N-carboxylatomethyl)-10-(N-1-methyl-3,6-diaza-2,5,8-trioxooctan-1,8-diyl)-1,4,7,10-tetraazacyclododecan, Gd-Komplex}-10-(N-2H,2H,4H,4H,5H,5H-3-oxa-perfluor-tridecanoyl)-1,4,7,10-tetraazacyclododecan, 1,4,7-Tris{1,4,7-tris[(N-carboxylatomethyl)]-10-[N-1-methyl-3-aza-2,5-dioxopentam-1,5-diyl]-1,4,7,10-tetraazacyclododecan, Gd-Komplex}-10-[2-(N-ethyl-N-perfluoroctylsulfonyl)-amino]-acetyl-1,4,7,10-tetraazacyclododecan, der Gadolinium-Komplex von 10-[2-Hydroxy-4-aza-5-oxo-7-aza-7(perfluoroctylsulfonyl)-nonyl]-1,4,7-tris(carboxymethyl)-1,4,7,10-tetraazacyclododecan,
1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex,
1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex,
1,4,7-Tris(carboxylatomethyl)-10-{(3-aza-4-oxo-hexan-5-yl)-säure-[N-3,6,9,12,15-pentaoxa)-hexadexyl)-N-(1H, 1H, 2H, 2H, 4H, 4H, 5H, 5H-3-oxa)-perfluortridecyl]-amid}-1,4,7,10-tetraazacyclododecan, Gadoliniumkomplex,
sowie 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex.

**14.** Formulierung gemäß Anspruch 3, dadurch gekennzeichnet, daß die weiteren perfluoralkylhaltigen Substanzen Verbindungen der allgemeinen Formel XV sind:

$$R_f — L^2 — G^1 \qquad (XXXII)$$

worin $R_f$ einen geradkettigen oder verzweigten Perfluoralkylrest mit 4 bis 30 Kohlenstoffatomen darstellt, $L^2$ für einen Linker und $G^1$ für eine hydrophile Gruppe steht.

**15.** Formulierung gemäß Anspruch 14, dadurch gekennzeichnet, daß der Linker $L^2$ eine direkte Bindung, eine —$SO_2$-Gruppe oder eine geradkettige oder verzweigte Kohlenstoffkette mit bis zu 20 Kohlenstoffatomen, welche mit ein oder mehreren — OH, -$COO^-$, -$SO_3$-Gruppen substituiert sein kann und/oder gegebenenfalls ein oder mehrere —O-, -S-, -CO-, -CONH-, -NHCO-, -CONR-, -NRCO-, -$SO_2$-, -$PO_4^-$-, -NH-, -NR-Gruppen, einen Arylring oder ein Piperazin enthält, wobei R für einen $C_1$- bis $C_{20}$-Alkylrest steht, welcher wiederum ein oder mehrere O-Atome enthalten kann und/oder mit —$COO^-$ oder $SO_3$-Gruppen substituiert sein kann.

**16.** Formulierung gemäß Anspruch 14, dadurch gekennzeichnet, daß die hydrophile Gruppe $G^1$ für ein Mono- oder Disaccharid, eine oder mehrere benachbarte —$COO^-$oder —$SO_3$-Gruppen, eine Dicarbonsäure, eine Isophthalsäure, eine Picolinsäure, eine Benzolsulfonsäure, eine Tetrahydropyrandicarbonsäure, eine 2,6-Pyridindicarbonsäure, ein quartäres Ammoniumion, eine Aminopolycarbonsäure, eine Aminodipolyethylenglycolsulfonsäure, eine Aminopolyethylenglycolgruppe, eine $SO_2$-$(CH_2)_2$-OH-Gruppe, eine Polyhydroxyalkylkette mit mindestens zwei Hydroxylgruppen oder eine oder mehrere Polyethylenglycolketten mit mindestens zwei Glycoleinheiten steht, wobei die Polyethylenglycolketten durch eine —OH oder — $OCH_3$-Gruppe terminiert sind.

**17.** Formulierung gemäß Anspruch 1, dadurch gekennzeichnet, daß die Perfluoralkylketten der perfluoralkylhaltigen Farbstoffmoleküle und der anderen perfluoralkylhaltigen Verbindungen 6 bis 12 Kohlenstoffatome enthalten.

**18.** Formulierung gemäß Anspruch 17, dadurch gekennzeichnet, daß die Perfluoralkylketten jeweils 8 Kohlenstoffatome enthalten.

**19.** Substanzen der allgemeinen Formel I

$$R_f — L — A \qquad (I)$$

worin $R_f$ einen geradkettigen oder verzweigten Perfluoralkylrest mit 4 bis 30 Kohlenstoffatomen darstellt, L für einen Linker und A für ein Farbstoffmolekül aus der Klasse der Polymethinfarbstoffe, Xanthinfarbstoffe oder der heteroaromatischen, kationischen Farbstoffe steht.

**20.** Substanzen gemäß Anspruch 19, dadurch gekennzeichnet, daß der Linker L eine direkte Bindung oder eine geradkettige oder verzweigte Kohlenstoffkette mit bis zu 20 Kohlenstoffatomen darstellt, welche mit ein oder mehreren —OH, -COOH, $-SO_3$-Gruppen substituiert sein kann und/oder gegebenenfalls durch eine oder mehrere —O-, -S-, -CO-, -CONH-, -NHCO-, -CONR-, -NRCO-, $-SO_2$-, -NH-, -NR-Gruppen oder ein Piperazin unterbrochen ist, wobei R für einen $C_1$- bis $C_{10}$-Alkylrest steht, welcher gegebenenfalls mit einer oder mehreren OH-Gruppen substituiert ist.

**21.** Substanzen gemäß Anspruch 19, dadurch gekennzeichnet, daß das Farbstoffmolekül A ein Cyaninfarbstoff, Sqariliumfarbstoff, Croconiumfarbstoff, Oxonolfarbstoff, Merocyaninfarbstoff, Kryptocyaninfarbstoff, Fluoresceinfarbstoff, Rhodaminfarbstoff, Oxazinfarbstoff, Phenoxazinfarbstoff, Thiazinfarbstoff oder Phenothiazinfarbstoff ist.

**22.** Substanzen gemäß Anspruch 22, dadurch gekennzeichnet, daß das Farbstoffmolekül A ein Molekül gemäß Formel II ist:

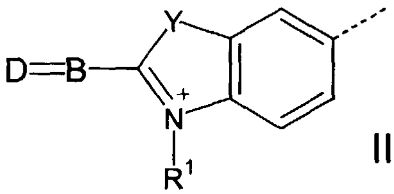

II

worin

D für ein Fragment entsprechend den allgemeinen Formeln III bis VI steht, wobei die mit einem Stern gekennzeichnete Position die Verknüpfung mit B bedeutet:

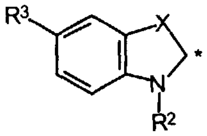

III

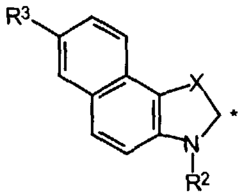

IV

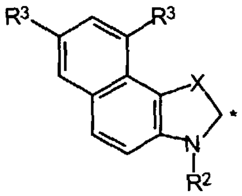

V

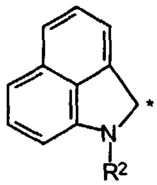

VI

und worin B für ein Fragment entsprechend den allgemeinen Formeln VII bis XII steht:

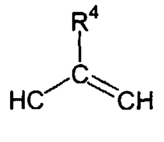

VII

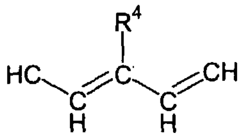

VIII

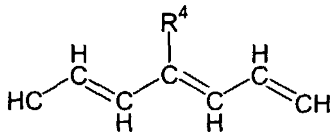

IX

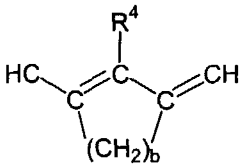

X

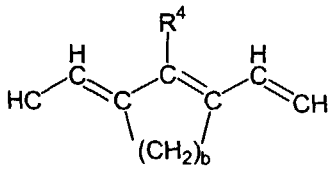

XI

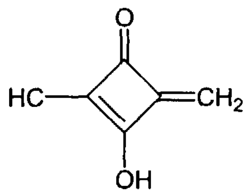

XII

wobei $R^1$ und $R^2$ eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$- $C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

$R^3$ für einen Rest -$COOE^1$, -$CONE^1E^2$, -$NHCOE^1$, -$NHCONHE^1$, -$NE^1E^2$, - $OE^1$, -$OSO_3E^1$, -$SO_3E^1$, -$SO_2NHE^1$, -$E^1$ steht,

wobei $E^1$ und $E^2$ unabhängig voneinander ein Wasserstoffatom, eine $C_1$-$C_4$-Sulfoalkylkette, eine gesättigte oder ungesättigte, verzweigte oder geradkettige $C_1$-$C_{50}$-Alkylkette darstellen, wobei die Kette oder Teile dieser Kette gegebenenfalls eine oder mehrere aromatische oder gesättigte zyklische $C_5$-$C_6$- oder bizyklische $C_{10}$-Einheiten formen können, und wobei die $C_1$- $C_{50}$-Alkylkette gegebenenfalls von 0 bis 15 Sauerstoffatomen und/oder von 0 bis 3 Carbonylgruppen unterbrochen ist und/oder mit 0 bis 5 Hydroxygruppen substituiert ist,

und wobei $R^4$ für ein Wasserstoffatom, ein Fluor-, Chlor-, Brom-, Iodatom oder eine verzweigte oder geradkettige $C_1$-$C_{10}$-Alkylkette steht,

b eine Zahl 2 oder 3 bedeutet,

sowie X und Y unabhängig voneinander O, S, -CH=CH- oder $C(CH_3)_2$ bedeuten.

**23.** Verfahren zur Herstellung von galenischen Formulierungen gemäß Anspruch 1, dadurch gekennzeichnet, daß die perfluoralkylhaltigen Farbstoffmoleküle und die anderen perfluoralkylhaltigen Verbindungen in einem Lösungsmittel unter starkem Rühren gelöst werden.

**24.** Verfahren zur Herstellung von galenischen Formulierungen gemäß Anspruch 1, dadurch gekennzeichnet, daß die perfluoralkylhaltigen Farbstoffmoleküle und die anderen perfluoralkylhaltigen Verbindungen in einem Lösungsmittel unter gleichzeitiger Behandlung mit Ultraschall gelöst werden.

**25.** Verfahren zur Herstellung von galenischen Formulierungen gemäß Anspruch 1, dadurch gekennzeichnet, daß die perfluoralkylhaltigen Farbstoffmoleküle und die anderen perfluoralkylhaltigen Verbindungen in einem Lösungsmittel unter gleichzeitiger Behandlung mit Mikrowellen gelöst werden.

**26.** Verfahren zur Herstellung von galenischen Formulierungen gemäß Anspruch 1, dadurch gekennzeichnet, daß die perfluoralkylhaltigen Farbstoffmoleküle in einem Lösungsmittel gelöst werden, die anderen perfluoralkylhaltigen

Verbindungen in einem anderen Lösungsmittel gelöst werden, beide Lösungen zusammengegeben werden und eines der beiden Lösungsmittel abdestilliert wird.

**27.** Feste Formulierung gemäß Anspruch 1, dadurch gekennzeichnet, daß sie durch Gefriertrocknen einer Lösung hergestellt wird, welche perfluoralkylhaltige Farbstoffmoleküle und andere perfluoralkylhaltige Substanzen enthält.

**28.** Verwendung von galenischen Formulierungen gemäß Anspruch 1 zur Herstellung von Kontrastmitteln für die optische Diagnostik, die Fluoreszenzdiagnostik, die Nahinfrarotdiagnostik, die Kernspintomographie, die Bildgebung unter Verwendung von Röntgenstrahlen (Röntgen oder Computertomographie) sowie für die Ultraschallbildgebung.

**29.** Verwendung von galenischen Formulierungen gemäß Anspruch 1 zur Herstellung von Kontrastmitteln zur Darstellung der Lymphknoten oder des blood-pools.

**30.** Verwendung von galenischen Formulierungen gemäß Anspruch 1 zur Herstellung von Mitteln für die intraoperative Diagnostik.

Fig. 1

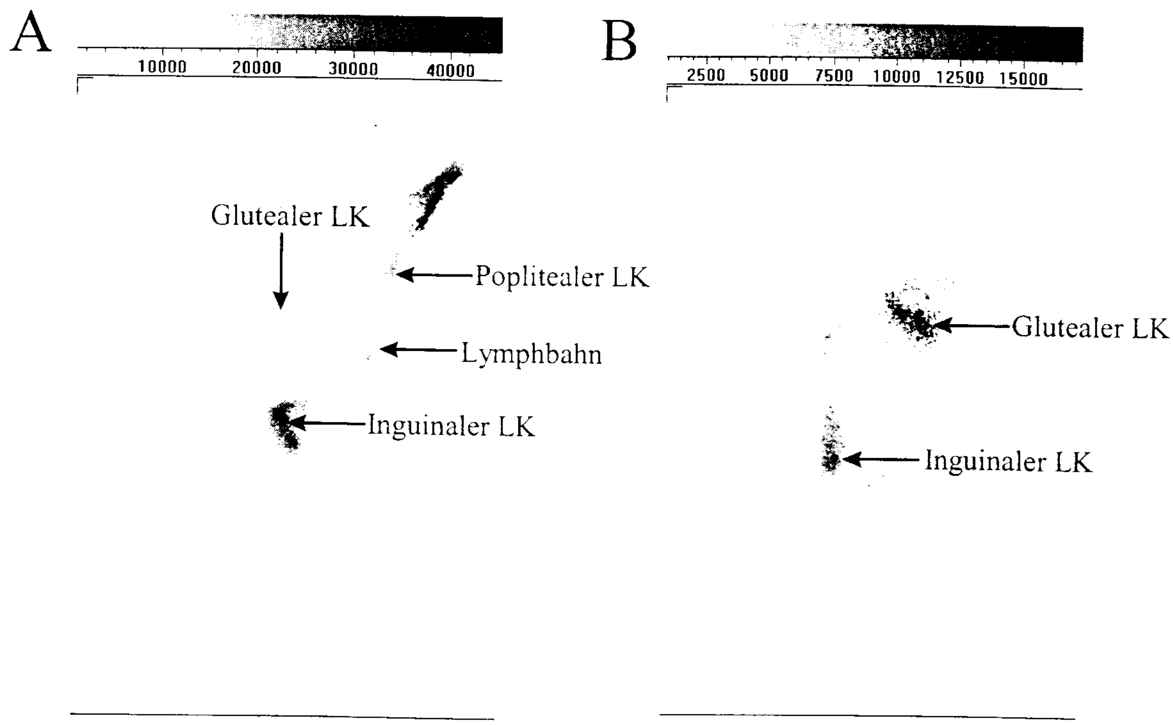
A
10000
20000
30000
40000
Glutealer LK
Popliteaer LK
Lymphbahn
Inguinaler LK
B
2500
5000
7500
10000
12500
15000
Glutealer LK
Inguinaler LK

Fig. 2

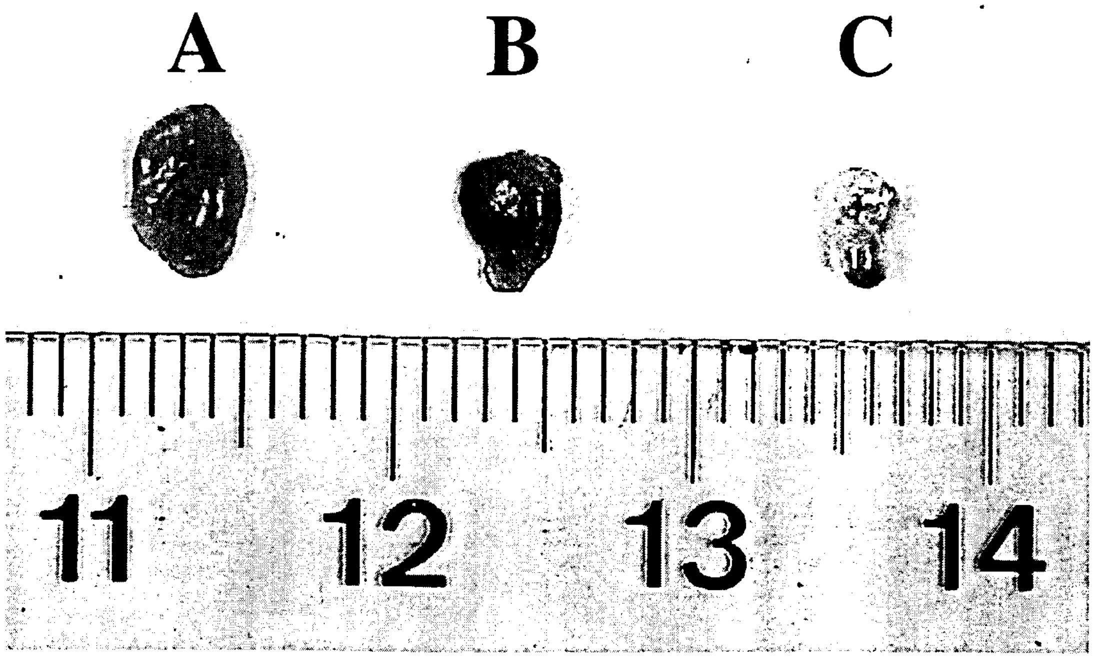

Fig. 3

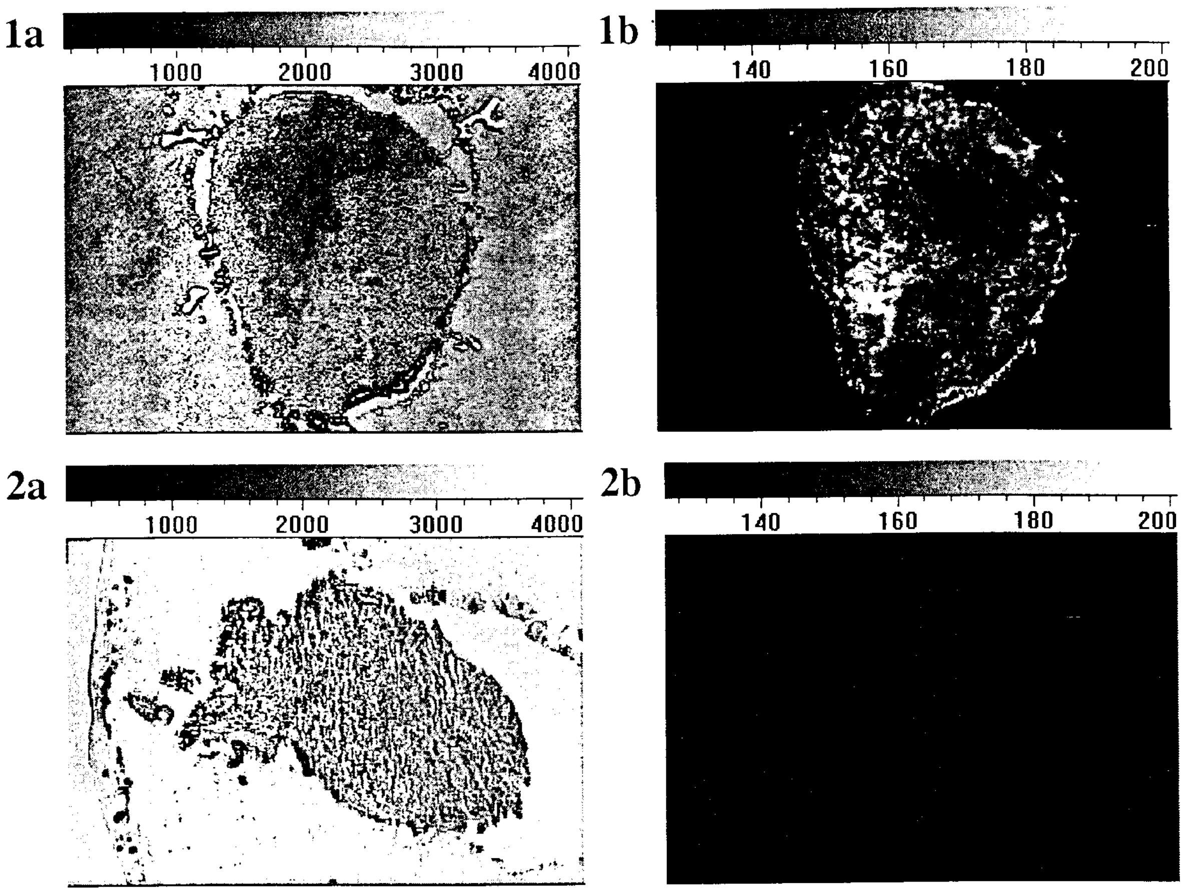
1a
1000
2000
3000
4000
1b
140
160
180
200
2a
1000
2000
3000
4000
2b
140
160
180
200

Fig. 4:

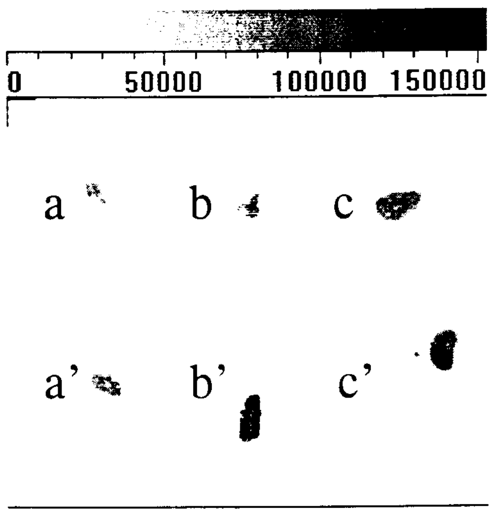

**Fig. 5:**

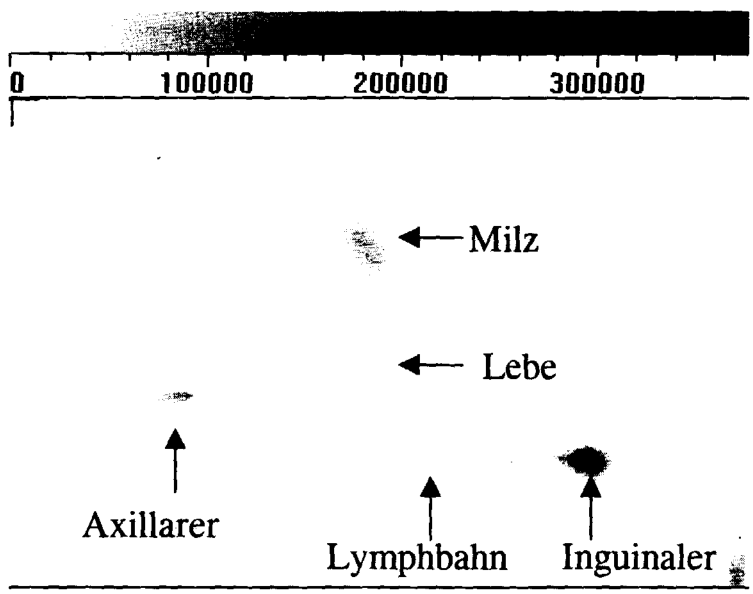
0
100000
200000
300000
Milz
Lebe
Axillarer
Lymphbahn
Inguinaler

Fig. 6:

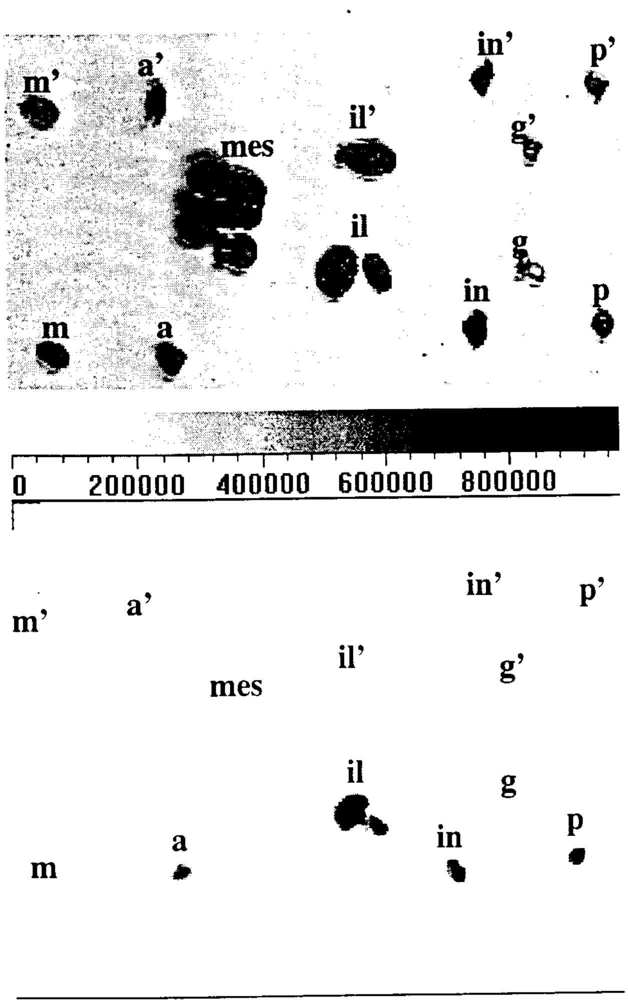
in'
p'
m'
a'
il'
g'
mes
il
g
in
p
m
a
0
200000
400000
600000
800000
in'
p'
m'
a'
il'
g'
mes
il
g
a
in
p
m